(19) Europäisches Patentamt
European Patent Office
Office européen des brevets

(11) EP 1 095 029 B1

(12) **FASCICULE DE BREVET EUROPEEN**

(45) Date de publication et mention de la délivrance du brevet:
**18.09.2002 Bulletin 2002/38**

(21) Numéro de dépôt: **99929400.2**

(22) Date de dépôt: **05.07.1999**

(51) Int Cl.[7]: **C07D 241/04**, A61K 31/495

(86) Numéro de dépôt international:
**PCT/FR99/01615**

(87) Numéro de publication internationale:
**WO 00/001677 (13.01.2000 Gazette 2000/02)**

(54) **DERIVES DE LA PIPERAZINE POUR L'INHIBITION DE LA REPLICATION DU VIRUS DE L'IMMUNODEFICIENCE HUMAINE**

PIPERAZINDERIVATE ZUR REPLIKATIONSHEMMUNG VON MENSCHLICHEM IMMUNDEFIZIENZ VIRUS

PIPERAZINE DERIVATIVES INHIBITING HUMAN IMMUNODEFICIENCY VIRUS REPLICATION

(84) Etats contractants désignés:
**AT BE CH CY DE DK ES FI FR GB GR IE IT LI LU MC NL PT SE**

(30) Priorité: **06.07.1998 FR 9808600**

(43) Date de publication de la demande:
**02.05.2001 Bulletin 2001/18**

(73) Titulaires:
- **UNIVERSITE PARIS 7 - Denis DIDEROT F-75005 Paris (FR)**
- **COMMISSARIAT A L'ENERGIE ATOMIQUE 75015 Paris (FR)**

(72) Inventeurs:
- **HEYMANS, Françoise F-75019 Paris (FR)**
- **DEREUDDRE-BOSQUET, Nathalie F-75016 Paris (FR)**
- **GODFROID, Jean-Jacques F-75020 Paris (FR)**
- **LAMOURI, Aazdine F-93150 Le Blanc Mesnil (FR)**
- **CLAYETTE, Pascal F-78000 Versailles (FR)**
- **MARTIN, Marc F-78250 Meulan (FR)**

(74) Mandataire: **Vialle-Presles, Marie José et al
Cabinet Orès,
6, Avenue de Messine
75008 Paris (FR)**

(56) Documents cités:
**EP-A- 0 368 670**
**WO-A-95/16688**
**FR-A- 2 637 499**
**FR-A- 2 637 592**
**FR-A- 2 645 859**

- **HEYMANS, FRANCOISE ET AL: "Design and modeling of new platelet -activating factor antagonists. 3. Relative importance of hydrophobicity and electronic distribution in piperazinic series." J. LIPID MEDIATORS CELL SIGNALLING (1997), 15(2), 161-173 CODEN: JLMSEO;ISSN: 0929-7855, XP002102680**
- **HEYMANS, F. ET AL: "Design and modeling of new PAF antagonists: 1,4-bis-(3',4',5'-trimethoxybenzoyl)-2-substituted carbonyloxymethylpiperazines" J. LIPID MEDIATORS CELL SIGNALLING (1994), 10(1-2), 153-4 CODEN: JLMSEO;ISSN: 0929-7855, XP002102681**
- **LAMOURI, AAZDINE ET AL: "Design and modeling of new platelet -activating factor antagonists. 1. Synthesis and biological activity of 1,4-bis(3',4',5'-trimethoxybenzoyl)-2-[[(s ubstituted carbonyl and carbamoyl)oxy]methyl]piperazines" J. MED. CHEM. (1993), 36(8), 990-1000 CODEN: JMCMAR;ISSN: 0022-2623, XP002102682**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 095 029 B1

## Description

L'Invention se rapporte à des dérivés de la pipérazine pour l'inhibition de la réplication du Virus de l'Immunodéficience Humaine.

L'infection par le Virus de l'Immunodéficience Humaine (VIH), qui a pour cibles cellulaires majeures les lymphocytes T CD4+ et les cellules de la lignée macrophagique, outre d'induire un effondrement des défenses immunitaires se traduisant chez les patients infectés par la survenue de maladies infectieuses opportunistes graves, est responsable d'un syndrome inflammatoire chronique et ce, tout particulièrement au niveau du système nerveux central, qui se manifeste par des complications neurologiques que l'on regroupe sous le nom d'encéphalopathie du VIH ou d'encéphalite subaiguë. Le « Platelet Activating Factor » (PAF), qui a été identifié comme étant un médiateur précoce de l'inflammation et de l'allergie, apparaît jouer un rôle essentiel dans ce syndrome inflammatoire, non seulement en raison de son rôle de médiateur inflammatoire mais également par sa neurotoxicité.

L'efficacité d'un traitement dans l'infection par le VIH est mesurée par la diminution de la charge virale dans le sang des patients infectés et par la restauration du taux de lymphocytes T CD4+ circulants. Par rapport à ces critères, deux classes d'antirétroviraux agissant à deux étapes différentes de la réplication virale, se sont révélées à ce jour être efficaces et sont, de ce fait, à la base du traitement actuel de l'infection par le VIH. Il s'agit :

- _ d'une part, des inhibiteurs de la transcriptase inverse qui visent à inhiber l'activité de l'enzyme virale assurant la rétrotranscription de l'acide ribonucléique (ARN) viral en acide désoxyribonucléique (ADN), forme sous laquelle le virus passe dans le noyau de la cellule infectée et s'intègre dans son génome cellulaire. Ces inhibiteurs de la transcriptase inverse sont représentés par la zidovudine (AZT), la didanosine (ddI), la zalcitabine (ddC), la lamivudine (3TC), la stavudine (d4T) et la névirapine ; et
- _ d'autre part, des inhibiteurs de la protéase tels que l'indinavir, le ritonavir, le saquinavir et le nelfinavir, qui agissent en inhibant l'activité d'une enzyme virale impliquée dans le processus de maturation des particules virales issues de la réplication - c'est-à-dire dans la mise en place de leurs différentes protéines internes et enzymes -, processus de maturation qui précède le relargage de ces particules vers d'autres cellules à infecter.

Ces antirétroviraux sont le plus souvent utilisées en combinaison : ainsi, la trithérapie, qui consiste à associer deux inhibiteurs de la transcriptase inverse et un inhibiteur de la protéase, est désormais prescrite en première intention et des protocoles de quadrithérapie sont actuellement en cours d'évaluation clinique.

En dépit de leur intérêt, les antirétroviraux actuellement disponibles n'ont pas vraiment résolu le problème de l'infection par le VIH.

En effet, d'une part, ils induisent des mutations virales qui sont responsables de l'apparition de résistances au traitement et, à terme, d'un phénomène d'échappement thérapeutique entraînant une reprise de l'infection. De plus, les résistances induites étant le plus souvent croisées entre les différents antirétroviraux, notamment entre les inhibiteurs de la protéase, les substitutions thérapeutiques sont très restreintes.

D'autre part, certains de ces antirétroviraux ont une diffusion tissulaire limitée. En particulier, ils sont incapables de traverser la barrière hématoméningée et d'atteindre, par conséquent, le système nerveux central qui constitue pourtant une cible privilégiée de l'infection virale.

Ces antirétroviraux ont, par ailleurs, de nombreux effets secondaires (anémie, leucopénie, nausées, vomissements, diarrhées, myalgies, céphalées, neuropathie périphérique, éruptions cutanées, fièvre, pancréatite, hépatotoxicité, ...) et ce, d'autant plus qu'ils sont utilisés en combinaison. Ces effets secondaires, outre d'être susceptibles d'entraîner une inobservance thérapeutique de par la gêne qu'ils procurent aux patients, peuvent dans certains cas imposer l'arrêt du traitement en raison de leur gravité.

Enfin, ces antirétroviraux présentent l'inconvénient d'avoir une action limitée au virus lui-même et d'être incapables de protéger ou de reconstruire le système immunitaire des patients infectés.

Le problème se pose, par conséquent, de fournir des composés qui, tout en étant actifs sur le VIH et aptes, par conséquent, à traiter efficacement une infection par ce virus, aient une large diffusion dans les tissus et soient notamment capables d'atteindre le système nerveux central, n'induisent pas de résistance et ne présentent pas de résistance croisée avec les antiviraux actuellement utilisés, permettent, non seulement d'éliminer le virus, mais également de protéger, voire de restaurer le système immunitaire, et présentent, en outre, une tolérance satisfaisante compatible avec une observance thérapeutique optimale.

Des dérivés de la 1,4,-di-(3',4',5'-triméthoxybenzoyl)-2-(N,N-méthoxycarbonyl-pipérazine ont été décrits dans la Demande de Brevet Français n° 89 13258 en tant que anti-ischémiques et anti-inflammatoires.

Par ailleurs, il a été montré, dans les Demandes de Brevets Français n° 89 13259 et n° 90 04798 ainsi que dans la Demande de Brevet Européen n° 0 368 670, que des dérivés trisubstitués de la pipérazine sont capables d'inhiber *in vitro* les effets du PAF sur l'agrégation des plaquettes sanguines et sont, à ce titre, susceptibles d'être utilisés dans le traitement de pathologies dans lesquelles ce médiateur apparaît impliqué et, notamment, dans le traitement des syndromes inflammatoires et des syndromes allergiques (inflammations aiguës, ulcérations gastro-intestinales, asthme, anaphylaxies cardiaques, ...).

L'intérêt de ce type de dérivés pipéraziniques en tant qu'antagonistes du PAF a été ultérieurement confirmé par les travaux de LAMOURI et *al.* (*J. Med. Chem.,* 1993, 36, 8, 990-1000) et de HEYMANS et *al.* (*J. Lipid Mediators Cell Signalling*, 1994, 10, 153-154 ; *J. Lipid Mediators Cell Signalling*, 1996, 15, 161-173) visant à préciser l'influence de la structure chimique de ces composés sur leur activité biologique.

Or, les Inventeurs, poursuivant leurs études sur les dérivés de la pipérazine, ont découvert que, de manière surprenante, certains de ces dérivés, outre d'être doués d'une activité anti-PAF, sont capables d'inhiber très efficacement la réplication du VIH, et sont donc propres à constituer une thérapeutique de choix dans le traitement de l'infection par le VIH puisqu'ils permettent non seulement de bloquer la multiplication de ce virus au sein de l'organisme et à terme son élimination, mais également de restaurer les fonctions immunitaires grâce à leur activité inhibitrice vis-à-vis du PAF.

La présente Invention a donc pour objet l'utilisation d'un dérivé de la pipérazine répondant à la formule générale (I) :

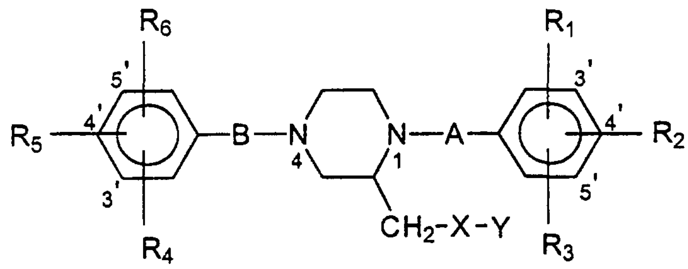

(I)

dans laquelle :

- A et B représentent, indépendamment l'un de l'autre, un groupe C=O, C=S ou $CR_7R_8$ dans lequel $R_7$ représente un atome d'hydrogène ou un groupe choisi parmi les groupes méthyle, cyano, cyanométhyle, $CO_2CH_3$ et (C=O)$CH_3$, tandis que $R_8$ représente un atome d'hydrogène ou un groupe phényle ;
- $R_1$, $R_2$, $R_3$, $R_4$, $R_5$ et $R_6$ représentent, indépendamment les uns des autres, un atome d'hydrogène, un groupe hydroxyle ou un groupe alkoxy en $C_1$ à $C_5$ linéaire ou ramifié ;
- X représente :
  - soit un groupe choisi parmi les groupes C=O, O(C=O), O(C=S), O($SO_2$), NH(C=O), NH(C=S), NH($SO_2$), S(C=O) et S(C=S), auquel cas Y représente soit un groupe $NR_9R_{10}$ ou $CR_9R_{10}R_{11}$ dans lequel $R_9$, $R_{10}$ et $R_{11}$ représentent, indépendamment les uns des autres, un atome d'hydrogène, un groupe alkyle en $C_1$ à $C_5$, alcényle en $C_2$ à $C_5$ ou alcynyle en $C_2$ à $C_5$ linéaire ou ramifié, soit un hétérocycle azoté comprenant de 5 à 10 atomes et éventuellement un ou plusieurs autres hétéroatomes choisis parmi l'azote, l'oxygène et le soufre ;
  - soit un atome d'oxygène ou de soufre ou un groupe choisi parmi les groupes O(C=O)O, NH(C=O)O et S(C=O)O, auquel cas Y représente un groupe $CR_9R_{10}R_{11}$ dans lequel $R_9$, $R_{10}$ et $R_{11}$ ont la même signification que précédemment ;

ou de l'un de ses sels pharmaceutiquement acceptables, pour la préparation d'un médicament pour inhiber la réplication du Virus de l'Immunodéficience Humaine (VIH).

Dans ce qui précède et ce qui suit :

- l'expression *« groupe alkyle en $C_1$ à $C_5$ linéaire ou ramifié »* désigne tout groupe hydrocarboné comprenant au plus 5 atomes de carbone tel qu'un groupe méthyle, éthyle, propyle, isopropyle, butyle, isobutyle, tertiobutyle ou pentyle ;
- l'expression *« groupe alcényle en $C_2$ à $C_5$ linéaire ou ramifié»* désigne tout groupe hydrocarboné comprenant de 2 à 5 atomes de carbone et une ou plusieurs doubles liaisons carbone-carbone tel qu'un groupe vinyle, allyle, butènyle, butadiényle ou pentènyle ;
- l'expression « *groupe alcynyle en $C_2$ à C linéaire ou ramifié »* désigne tout groupe hydrocarboné comprenant de

2 à 5 atomes de carbone et une ou plusieurs triples liaisons carbone-carbone tel qu'un groupe éthynyle, propynyle, butynyle ou pentynyle ;

- l'expression « *groupe alkoxy en $C_1$ à $C_5$ linéaire ou ramifié* » désigne tout groupe de formule OR dans laquelle R représente une chaîne hydrocarbonée, saturée ou insaturée, comprenant au plus 5 atomes de carbone tel qu'un groupe méthoxy, éthoxy, propoxy, isopropoxy, butoxy, tertiobutoxy, pentoxy, allyloxy, butènyloxy, méthylpropènyloxy, pentènyloxy, méthylbutènyloxy ou encore pentadiènyloxy, tandis que
- l'expression « *hétérocycle azoté comprenant de 5 à 10 atomes et éventuellement un ou plusieurs autres hétéroatomes choisis parmi l'azote, l'oxygène et le soufre »* désigne tout cycle, saturé ou insaturé, comprenant de 5 à 10 atomes dont l'un au moins est un atome d'azote et comportant éventuellement un ou plusieurs autres atomes choisis parmi l'azote, l'oxygène et le soufre tel qu'un groupement pyrrolyle, imidazolyle, pyrazolyle, triazolyle, tétrazolyle, pyrrolidinyle, pyrazolidinyle, imidazolydinyle, pipéridinyle ou indolyle.

Conformément à l'Invention, $R_1$, $R_2$ et $R_3$ sont portés de préférence par les atomes de carbone situés en position 3', 4' et 5' du noyau phényle lié au groupe A, tandis que $R_4$, $R_5$ et $R_6$ sont portés de préférence par les atomes de carbone situés en position 3', 4' et 5' du noyau phényle lié au groupe B.

Selon une première disposition préférée de l'Invention, le dérivé de la pipérazine répond à la formule particulière (I-a) :

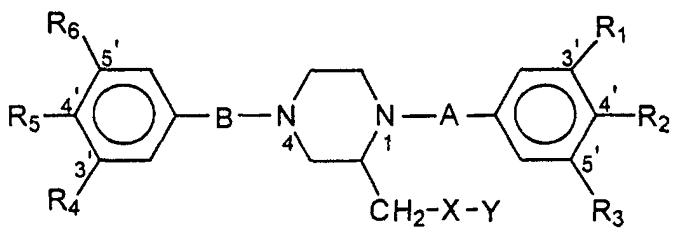

(I-a)

dans laquelle A et B représentent, indépendamment l'un de l'autre, un groupe C=O ou C=S, tandis que $R_1$, $R_2$, $R_3$, $R_4$, $R_5$, $R_6$, X et Y ont la même signification que dans la formule générale (I) définie ci-avant.

De manière avantageuse, dans la formule particulière (I-a), $R_1$, $R_2$, $R_4$ et $R_5$ représentent un groupe méthoxy, $R_3$ et $R_6$ représentent tous deux un atome d'hydrogène ou un groupe méthoxy, X représente :

- soit un groupe O(C=O) ou NH(C=O), auquel cas Y représente un groupe $NR_9R_{10}$ ou $CR_9R_{10}R_{11}$ dans lequel $R_9$, $R_{10}$ et $R_{11}$ ont la même signification que dans la formule générale (I) ci-avant, ou bien un hétérocycle azoté comprenant de 5 à 10 atomes et éventuellement un ou plusieurs autres hétéroatomes choisis parmi l'azote, l'oxygène et le soufre ;
- soit un groupe NH(C=O)O, auquel cas Y représente un groupe $CR_9R_{10}R_{11}$ dans lequel $R_9$, $R_{10}$ et $R_{11}$ ont également la même signification que dans la formule générale (I) ci-avant.

De manière préférée, le dérivé de la pipérazine répondant à la formule particulière (I-a) est choisi parmi la 1,4-di-(3',4',5'-triméthoxybenzoyl)-2-(N,N-diéthylaminocarbonyloxyméthyl)-pipérazine, la 1,4-di-(3',4',5'-triméthoxybenzoyl)-2-(N,N-dipropylaminocarbonyloxyméthyl)-pipérazine, la 1,4-di-(3',4',5'-triméthoxybenzoyl)-2-(1'-pipéridinocarbonyloxyméthyl)-pipérazine, la 1,4-di-(3',4',5'-triméthoxybenzoyl)-2-(1'-pyrrolidinocarbonyloxyméthyl)-pipérazine, la 1,4-di-(3',4'-diméthoxybenzoyl)-2-(N,N-diéthylaminocarbonyloxyméthyl)-pipérazine, la 1,4-di-(3',4',5'-triméthoxybenzoyl)-2-(isopropyloxycarbonylaminométhyl)-pipérazine, la 1,4-di-(3',4',5'-triméthoxybenzoyl)-2-(tertiobutylcarbonylaminométhyl)-pipérazine, la 1-(3',4',5'-triméthoxybenzoyl)-4-(3',4',5'-triméthoxythiobenzoyl)-2-(N,N-diéthylaminocarbonyloxyméthyl)-pipérazine, la 1,4-di(3',4',5'-triméthoxythio-benzoyl)-2-(N,N-diéthylaminocarbonyloxyméthyl)-pipérazine et la 1-(3',4',5'-tri-méthoxythiobenzoyl)-4-(3',4',5'-triméthoxybenzoyl)-2-(N,N-diéthylamino-carbonyloxyméthyl)-pipérazine.

De manière particulièrement préférée, le dérivé de la pipérazine répondant à la formule particulière (I-a) est la 1,4-di-(3',4',5'-triméthoxybenzoyl)-2-(N,N-diéthylaminocarbonyloxyméthyl)-pipérazine.

Selon une autre disposition préférée de l'Invention, le dérivé de la pipérazine répond à la formule particulière (I-b) :

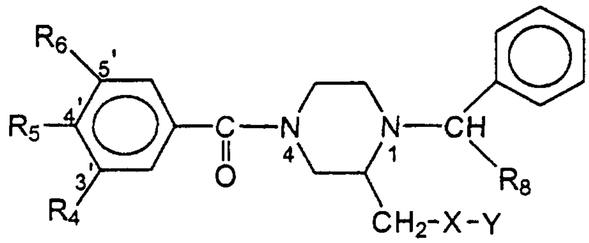

(I-b)

dans laquelle $R_8$ représente un atome d'hydrogène ou un groupe phényle, tandis que $R_4$, $R_5$, $R_6$, X et Y ont la même signification que dans la formule générale (I) définie ci-avant.

De manière avantageuse, dans la formule particulière (I-b), $R_4$, $R_5$ et $R_6$ représentent un groupe méthoxy, X représente un groupe O(C=O), tandis que Y représente un groupe $NR_9R_{10}$ dans lequel $R_9$ et $R_{10}$ représentent tous deux un groupe alkyle en $C_1$ à $C_5$, alcényle en $C_2$ à $C_5$ ou alcynyle en $C_2$ à $C_5$ linéaire ou ramifié, ou bien un hétérocycle azoté comprenant de 5 à 10 atomes et éventuellement un ou plusieurs autres hétéroatomes choisis parmi l'azote, l'oxygène et le soufre.

De manière préférée, le dérivé pipérazinique répondant à la formule particulière (I-b) est choisi parmi la 1-benzyl-4-(3',4',5'-triméthoxybenzoyl)-2-(N,N-diéthylaminocarbonyloxyméthyl)-pipérazine et la 1-diphénylméthyl-4-(3',4',5'-triméthoxybenzoyl)-2-(N,N-diéthylaminocarbonyloxyméthyl)-pipérazine.

Selon encore une autre disposition préférée de l'Invention, le dérivé de la pipérazine répond à la formule particulière (I-c) :

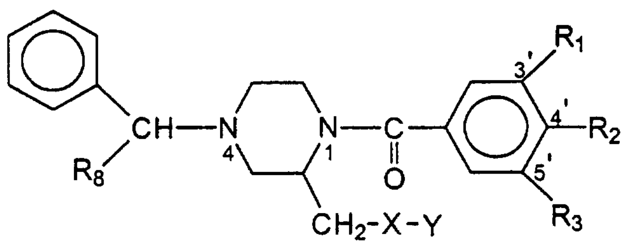

(I-c)

dans laquelle $R_8$ représente un atome d'hydrogène ou un groupe phényle, tandis que $R_1$, $R_2$, $R_3$, X et Y ont la même signification que dans la formule générale (I) définie ci-avant.

De manière avantageuse, dans la formule particulière (I-c), $R_1$, $R_2$ et $R_3$ représentent un groupe méthoxy, X représente un groupe O(C=O), tandis que Y représente un groupe $NR_9R_{10}$ dans lequel $R_9$ et $R_{10}$ représentent tous deux un groupe alkyle en $C_1$ à $C_5$, alcényle en $C_2$ à $C_5$ ou alcynyle en $C_2$ à $C_5$ linéaire ou ramifié, ou bien un hétérocycle azoté comprenant de 5 à 10 atomes et éventuellement un ou plusieurs autres hétéroatomes choisis parmi l'azote, l'oxygène et le soufre.

De manière préférée, le dérivé pipérazinique répondant à la formule particulière (I-c) est la 1-(3',4',5'-triméthoxybenzoyl)-4-benzyl-2-(N,N-diéthylaminocarbonyloxyméthyl)-pipérazine.

L'utilisation des dérivés de la pipérazine de formule générale (I) et de leurs sels pharmaceutiquement acceptables pour la préparation d'un médicament pour inhiber la réplication du VIH présente de nombreux avantages. En effet, outre de permettre une maîtrise de l'expansion du virus chez les patients infectés et à terme son élimination grâce à leur action antivirale, ils sont capables d'induire une reconstruction du système immunitaire de ces patients par leur action inhibitrice sur l'activité du PAF. Par ailleurs, bien que leur mécanisme d'action sur la réplication du VIH ne soit pas encore complètement élucidé, ces dérivés de la pipérazine apparaissent n'agir ni sur la transcriptase inverse, ni sur la protéase virale, en sorte que leur utilisation permet de s'affranchir des problèmes de résistances croisées auxquels se heurtent actuellement les cliniciens dans le traitement de l'infection par le VIH. En outre, les dérivés de la pipérazine utiles conformément à l'Invention font preuve d'une absence de cytotoxicité plaidant en faveur d'une tolérance très satisfaisante.

Les dérivés de la pipérazine de formule générale (I) et leurs sels pharmaceutiquement acceptables sont susceptibles d'être obtenus par un procédé qui comprend :

* lorsque le dérivé que l'on désire préparer répond à la formule générale (I) dans laquelle A et B sont tous deux un groupe C=O et lorsque les noyaux phényle qui leur sont liés portent les mêmes substituants :

  a) l'acylation d'un composé répondant à la formule générale (II) ci-après :

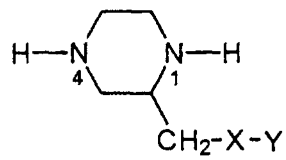

(II)

  dans laquelle X et Y ont la même signification que dans la formule générale (I) définie ci-avant, avec un réactif de formule générale (III) ci-après :

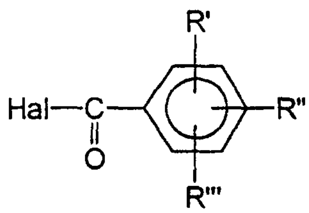

(III)

  dans laquelle Hal représente un atome d'halogène et, de préférence, un atome de chlore, tandis que R', R'' et R''' représentent, indépendamment les uns des autres un atome d'hydrogène, un groupe hydroxyle ou un groupe alkoxy en $C_1$ à $C_5$ linéaire ou ramifié, puis, si désiré
  b) la transformation du composé obtenu à l'étape a) en l'un de ses sels pharmaceutiquement acceptables ;

* lorsque le dérivé que l'on désire préparer répond à la formule générale (I) dans laquelle A et B sont tous deux un groupe C=O et lorsque les noyaux phényle qui leur sont liés portent des substituants différents :

  a) l'acylation d'un composé répondant à la formule générale (II) telle que définie ci-avant, avec un réactif de formule générale (III-a) ci-après, puis avec un réactif de formule générale (III-b) ci-après :

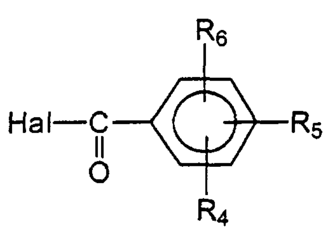

(III-a)

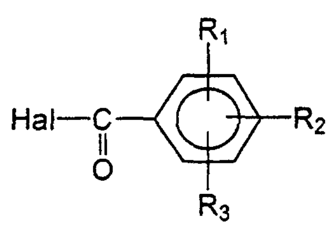

(III-b)

  dans lesquelles Hal représente un atome d'halogène et, de préférence, un atome de chlore, tandis que $R_1$, $R_2$, $R_3$, $R_4$, $R_5$ et $R_6$ ont la même signification que dans la formule générale (I) définie ci-avant, puis, si désiré
  b) la transformation du composé obtenu à l'étape a) en l'un de ses sels pharmaceutiquement acceptables ;

* lorsque le dérivé que l'on désire préparer répond à la formule générale (I) dans laquelle A et B sont tous deux un groupe C=S et lorsque les noyaux phényle qui leur sont liés portent les mêmes substituants :

  a) l'acylation d'un composé répondant à la formule générale (II) telle que définie ci-avant, avec un réactif de

formule générale (III) telle que définie ci-avant,
b) la réaction du composé obtenu à l'étape a) avec un réactif capable de transformer une fonction carbonyle en une fonction thiocarbonyle comme, par exemple, le réactif de LAWESSON, puis, si désiré
c) la transformation du composé obtenu à l'étape b) en l'un de ses sels pharmaceutiquement acceptables ;

* lorsque le dérivé que l'on désire préparer répond à la formule générale (I) dans laquelle l'un des deux groupes A et B est un groupe C=O, tandis que l'autre est un groupe C=S, et lorsque les noyaux phényle qui leur sont liés portent les mêmes substituants :

a) l'acylation d'un composé répondant à la formule générale (II) telle que définie ci-avant, avec un réactif de formule générale (III) telle que définie ci-avant, après avoir éventuellement protégé (par exemple par tritylation) l'atome d'azote situé en position 4 du cycle pipérazine de ce composé,
b) la réaction du composé obtenu à l'étape a) avec un réactif capable de transformer une fonction carbonyle en une fonction thiocarbonyle,
c) l'acylation du composé obtenu à l'étape b) avec un réactif de formule générale (III) telle que définie ci-avant, après déprotection (par exemple par détritylation) de l'atome d'azote situé en position 4 du cycle pipérazine si celui-ci a été protégé, puis, si désiré
d) la transformation du composé obtenu à l'étape c) en l'un de ses sels pharmaceutiquement acceptables ;

* lorsque le dérivé que l'on désire préparer répond à la formule générale (I) dans laquelle A est un groupe C=O, tandis que B est un groupe C=S, et lorsque les noyaux phényle qui leur sont liés portent des substituants différents :

a) l'acylation d'un composé répondant à la formule générale (II) telle que définie ci-avant, avec un réactif de formule générale (III-a) telle que définie ci-avant,
b) la réaction du composé obtenu à l'étape a) avec un réactif capable de transformer une fonction carbonyle en une fonction thiocarbonyle,
c) l'acylation du composé obtenu à l'étape b) avec un réactif de formule générale (III-b) telle que définie ci-avant, puis, si désiré
d) la transformation du composé obtenu à l'étape c) en l'un de ses sels pharmaceutiquement acceptables ;

* lorsque le dérivé que l'on désire préparer répond à la formule générale (I) dans laquelle A est un groupe C=S, tandis que B est un groupe C=O, et lorsque les noyaux phényle qui leur sont liés portent des substituants différents :

a) l'acylation d'un composé répondant à la formule générale (II) telle que définie ci-avant, avec un réactif de formule générale (III-b) telle que définie ci-avant, après avoir protégé l'atome d'azote situé en position 4 du cycle pipérazine de ce composé,
b) la réaction du composé obtenu à l'étape a) avec un réactif capable de transformer une fonction carbonyle en une fonction thiocarbonyle,
c) l'acylation du composé obtenu à l'étape b) avec un réactif de formule générale (III-b) telle que définie ci-avant, après déprotection de l'atome d'azote situé en position 4 du cycle pipérazine, puis, si désiré
d) la transformation du composé obtenu à l'étape c) en l'un de ses sels pharmaceutiquement acceptables ;

* lorsque le dérivé que l'on désire préparer répond à la formule générale (I) dans laquelle A et B sont tous deux un groupe $CR_7R_8$ et lorsque les noyaux phényle qui leur sont liés portent les mêmes substituants :

a) l'alkylation d'un composé répondant à la formule générale (II) telle que définie ci-avant, avec un réactif de formule générale (IV) ci-après :

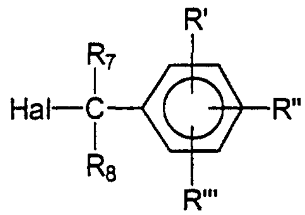

(IV)

dans laquelle Hal représente un atome d'halogène et, de préférence, un atome de chlore, $R_7$ et $R_8$ ont la même signification que dans la formule générale (I) définie ci-avant, tandis que R', R" et R"' représentent, indépendamment les uns des autres, un atome d'hydrogène, un groupe hydroxyle ou un groupe alkoxy en $C_1$ à $C_5$ linéaire ou ramifié, puis, si désiré

b) la transformation du composé obtenu à l'étape a) en l'un de ses sels pharmaceutiquement acceptables ;

* lorsque le dérivé que l'on désire préparer répond à la formule générale (I) dans laquelle A et B sont tous deux un groupe $CR_7R_8$ et lorsque les noyaux phényle qui leur sont liés portent des substituants différents :

a) l'alkylation d'un composé répondant à la formule générale (II) telle que définie ci-avant avec un réactif de formule générale (IV-a) ci-après, puis avec un réactif de formule générale (IV-b) ci-après :

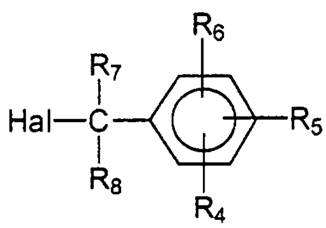

(IV-a)

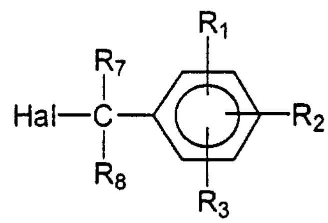

(IV-b)

dans lesquelles Hal représente un atome d'halogène et, de préférence, un atome de chlore, tandis que $R_1$, $R_2$, $R_3$, $R_4$, $R_5$ ,$R_6$, $R_7$ et $R_8$ ont la même signification que dans la formule générale (I) définie ci-avant ;

b) la transformation du composé obtenu à l'étape a) en l'un de ses sels pharmaceutiquement acceptables ;

* lorsque le dérivé que l'on désire préparer répond à la formule générale (I) dans laquelle l'un des deux groupes A et B est un groupe C=O, tandis que l'autre est un groupe $CR_7R_8$ :

a) l'acylation d'un composé répondant à la formule générale (II) telle définie ci-avant, avec un réactif de formule générale (III) telle que définie ci-avant, après avoir éventuellement protégé l'atome d'azote situé en position 4 du cycle pipérazine de ce composé,

b) l'alkylation du composé obtenu à l'étape a) avec un réactif de formule générale (IV) telle que définie ci-avant, après déprotection de l'atome d'azote situé en position 4 du cycle pipérazine lorsque celui-ci a été protégé, puis, si désiré

c) la transformation du composé obtenu à l'étape b) en l'un de ses sels pharmaceutiquement acceptables.

Les composés de formule générale (II) dans laquelle X représente un groupe O(C=O), peuvent être préparés à partir de la 1,4-dibenzyl-2-hydroxyméthylpipérazine (laquelle peut être obtenue par le procédé décrit par JUCKER et RISSI dans *Helv. Chem. Acta*, 1962, 45, 2383-2402) en acylant cette dernière par un halogénocarbonate d'aryle du type chlorocarbonate de phényle, puis en soumettant le composé résultant à une réaction de substitution par action d'un réactif de formule H-Y dans laquelle Y a la même signification que dans la formule générale (II), et, enfin, en soumettant le composé ainsi obtenu à une hydrogénolyse, par exemple au moyen de palladium sur charbon actif.

Les composés de formule générale (II) dans laquelle X représente un groupe O(C=S), peuvent également être préparés à partir de la 1,4-dibenzyl-2-hydroxy-méthylpipérazine, mais dans ce cas, cette dernière est soumise à une réaction d'addition-élimination, par exemple par action successive du thiophosgène et d'un réactif de formule H-Y dans laquelle Y a la même signification que dans la formule générale (II), puis on soumet le composé résultant à une débenzylation, par exemple au moyen de chloroformate de 2,2,2-trichloroéthyle, et en présence de zinc et d'acide acétique.

Les composés de formule générale (II) dans laquelle X représente un groupe $O(SO_2)$, peuvent aussi être obtenus à partir de la 1,4-dibenzyl-2-hydroxyméthylpipérazine en soumettant celle-ci à une réaction d'addition-élimination, par exemple par action successive du chlorure de sulfuryle et d'un réactif de formule H-Y dans laquelle Y a la même signification que dans la formule générale (II), puis en soumettant le composé résultant à une débenzylation.

Les composés de formule générale (II) dans laquelle X représente un groupe NH(C=O), NH(C=S) ou NH (C=O)O, peuvent être préparés à partir de la 1,4-dibenzyl-2-aminométhylpipérazine en faisant réagir cette dernière

avec un réactif choisi parmi les réactifs de formules Hal(C=O)Y, Hal(C=S)Y et Hal(C=O)O-Y dans lesquelles Hal représente un atome d'halogène et, de préférence, un atome de chlore, tandis que Y a la même signification que dans la formule générale (II), puis en soumettant le composé résultant à une hydrogénolyse, dans le cas où X représente un groupe NH(C=O) ou NH(C=O)O, et à une débenzylation, dans le cas où X représente un groupe NH(C=S).

Les composés de formule générale (II) dans laquelle X représente un groupe $NH(SO_2)$, peuvent également être préparés à partir de la 1,4-dibenzyl-2-aminométhylpipérazine, mais dans ce cas, celle-ci est soumise à une réaction d'addition-élimination par exemple, par action successive du chlorure de sulfuryle et d'un réactif de formule H-Y dans laquelle Y a la même signification que dans la formule générale (II), puis à une débenzylation.

La 1,4-dibenzyl-2-aminométhylpipérazine peut, elle-même, être obtenue en faisant réagir du 2,3-dibromopropionitrile avec de la N,N'-dibenzyléthylènediamine en présence de triéthylamine et de benzène, de manière à obtenir la 1,4-dibenzyl-2-cyanopipérazine, puis en soumettant cette dernière à une réaction de réduction, par exemple par action d'hydrure d'aluminium et de lithium.

Les composés de formule générale (II) dans laquelle X représente un groupe S(C=O), peuvent être préparés à partir de la 1,4-dibenzyl-2-thiométhylpipérazine en acylant cette dernière par un halogénocarbonate d'aryle du type chlorocarbonate de phényle, puis en soumettant le composé résultant à une réaction de substitution par action d'un réactif de formule H-Y dans laquelle Y a la même signification que dans la formule générale (II), et, enfin, en soumettant le composé ainsi obtenu à une débenzylation.

De manière similaire, les composés de formule générale (II) dans laquelle X représente un groupe S(C=S), peuvent être préparés à partir de la 1,4-dibenzyl-2-thiométhylpipérazine en soumettant cette dernière à une réaction d'addition-élimination, par exemple par action successive du thiophosgène et d'un réactif de formule H-Y dans laquelle Y a la même signification que dans la formule générale (II), et en soumettant le composé ainsi obtenu à une débenzylation.

La 1,4-dibenzyl-2-thiométhylpipérazine peut elle-même être obtenue à partir de la 1,4-dibenzyl-2-chlorométhylpipérazine en soumettant cette dernière à une réaction de substitution nucléophile, par exemple au moyen de thioacétate de sodium ou de potassium, puis en saponifiant le composé résultant par une base telle que la soude.

Les composés de formule générale (II) dans laquelle X représente un atome d'oxygène ou un atome de soufre, peuvent, quant à eux, être préparés respectivement à partir de la 1,4-dibenzyl-2-hydroxyméthylpipérazine et de la 1,4-dibenzyl-2-thiométhylpipérazine en soumettant celles-ci à une éthérification, par exemple par action d'une base, puis d'un réactif de formule Hal-Y dans laquelle Hal représente un atome d'halogène et, de préférence, un atome de chlore, tandis que Y a la même signification que dans la formule générale (II), puis en soumettant le composé ainsi obtenu à une hydrogénolyse.

Les composés de formule générale (II) dans lesquels X représentent soit un groupe O(C=O)O, soit un groupe S(C=O)O, peuvent être obtenus à partir de la 1,4-dibenzyl-2-hydroxyméthylpipérazine et de la 1,4-dibenzyl-2-thiométhylpipérazine respectivement en les soumettant à une réaction d'addition-élimination, par exemple par action d'un réactif de formule Hal(C=O)O-Y dans laquelle Hal représente un atome d'halogène et, de préférence, un atome de chlore, tandis que Y a la même signification que dans la formule générale (II), puis en soumettant les composés résultants à une débenzylation.

La présente Invention englobe, pour l'utilisation pour la préparation d'un médicament destiné à inhiber la réplication du VIH, à la fois des dérivés de la pipérazine connus et des dérivés de la pipérazine nouveaux en eux-mêmes.

La présente Invention a donc également pour objet de nouveaux dérivés de la pipérazine qui répondent à la formule générale (I) telle que définie ci-avant, à condition toutefois que, dans cette formule générale (I):

- dans le cas où $R_1$, $R_2$ et $R_3$ sont des groupes alkoxy en $C_1$ à $C_4$ saturés, linéaires ou ramifiés, et sont portés par les atomes de carbone situés en position 3', 4' et 5' du noyau phényle lié au groupe A, et où B est un groupe C=O, Y soit différent:
    - d'un groupe alkyle en $C_1$ à $C_4$, alcényle en $C_2$ à $C_4$ et alcynyle en $C_2$ à $C_4$ linéaire ou ramifié, lorsque X représente un atome d'oxygène ou de soufre ; et
    - d'un groupe alkyle en $C_1$ à $C_4$, alcényle en $C_2$ à $C_4$ et alcynyle en $C_2$ à $C_4$ linéaire ou ramifié, et d'une amine substituée ou non par un ou deux groupes alkyles en $C_1$ à $C_4$, alcényles en $C_2$ à $C_4$ et alcynyles en $C_2$ à $C_4$ linéaires ou ramifiés, lorsque X représente un groupe C=O ou O(C=O);
- dans le cas où $R_1$, $R_2$, $R_3$, $R_4$, $R_5$ et $R_6$ représentent tous un groupe méthoxy et où $R_1$, $R_2$, $R_3$ sont portés par les atomes de carbone situés en position 3', 4' et 5' du noyau phényle lié au groupe A, tandis que $R_4$, $R_5$ et $R_6$ sont portés par les atomes de carbone situés en position 3', 4' et 5' du noyau phényle lié au groupe B, Y soit différent :
    - d'un groupe alkyle en $C_5$ ou $C_6$ linéaire ou ramifié, d'une amine substituée par un groupe alkyle en $C_5$ linéaire

ou ramifié, et d'une amine substituée par deux groupes alkyles en $C_5$ linéaires, lorsque A et B représentent tous deux un groupe C=O et X représente un groupe O(C=O) ; et

- d'un groupe $CH(CH_2CH_3)_2$, lorsque A et B représentent tous deux un groupe $CH_2$ et X représente un groupe O(C=O);

ainsi que ses sels pharmaceutiquement acceptables.

Conformément à l'Invention, dans ces nouveaux dérivés pipéraziniques, $R_1$, $R_2$ et $R_3$ sont portés de préférence par les atomes de carbone situés en position 3', 4' et 5' du noyau phényle lié au groupe A, tandis que $R_4$, $R_5$ et $R_6$ sont portés de préférence par les atomes de carbone situés en position 3', 4' et 5' du noyau phényle lié au groupe B.

Selon un premier mode de réalisation préféré de ces nouveaux dérivés de la pipérazine, ces derniers répondent à la formule particulière (I-a) :

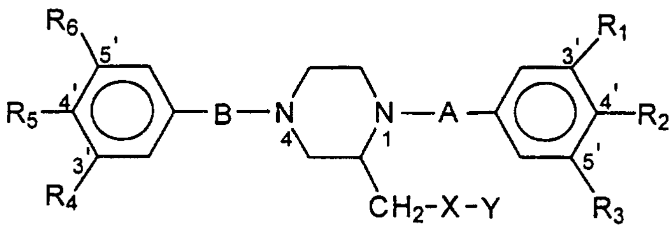

(I-a)

dans laquelle A et B représentent, indépendamment l'un de l'autre, un groupe C=O ou un groupe C=S, tandis que $R_1$, $R_2$, $R_3$, $R_4$, $R_5$, $R_6$, X et Y ont la même signification que dans la formule générale (I) définie ci-avant, à condition toutefois que :

- dans le cas où $R_1$, $R_2$ et $R_3$ sont des groupes alkoxy en $C_1$ à $C_4$ saturés, linéaires ou ramifiés, et où B est un groupe C=O, Y soit différent :

  - d'un groupe alkyle en $C_1$ à $C_4$, alcényle en $C_2$ à $C_4$ et alcynyle en $C_2$ à $C_4$ linéaire ou ramifié, lorsque X représente un atome d'oxygène ou de soufre ; et
  - d'un groupe alkyle en $C_1$ à $C_4$, alcényle en $C_2$ à $C_4$ et alcynyle en $C_2$ à $C_4$ linéaire ou ramifié, et d'une amine substituée ou non par un ou deux groupes alkyles en $C_1$ à $C_4$, alcényles en $C_2$ à $C_4$ et alcynyles en $C_2$ à $C_4$ linéaires ou ramifiés, lorsque X représente un groupe C=O ou O(C=O);

- dans le cas où $R_1$, $R_2$, $R_3$, $R_4$, $R_5$ et $R_6$ représentent tous un groupe méthoxy, Y soit différent d'un groupe alkyle en $C_5$ ou $C_6$ linéaire ou ramifié, d'une amine substituée par un groupe alkyle en $C_5$ linéaire ou ramifié, et d'une amine substituée par deux groupes alkyles en $C_5$ linéaires, lorsque X représente un groupe O(C=O).

Parmi les nouveaux dérivés pipéraziniques de formule particulière (I-a), on préfère notamment ceux dans lesquels $R_1$, $R_2$, $R_4$ et $R_5$ représentent un groupe méthoxy, $R_3$ et $R_6$ représentent tous deux un atome d'hydrogène ou un groupe méthoxy, et X représente :

- soit un groupe O(C=O), auquel cas Y représente un hétérocycle azoté comprenant de 5 à 10 atomes et éventuellement un ou plusieurs autres hétéroatomes choisis parmi l'azote, l'oxygène et le soufre, ou encore, lorsque $R_3$ et $R_6$ représentent un atome d'hydrogène, un groupe $NR_9R_{10}$ dans lequel $R_9$ et $R_{10}$ représentent tous deux un groupe alkyle en $C_1$ à $C_5$, alcényle en $C_2$ à $C_5$ ou alcynyle en $C_2$ à $C_5$ linéaire ou ramifié ;
- soit un groupe NH(C=O), auquel cas Y représente soit un groupe $NR_9R_{10}$ ou $CR_9R_{10}R_{11}$ dans lequel $R_9$, $R_{10}$ et $R_{11}$ représentent, indépendamment les uns des autres, un atome d'hydrogène ou un groupe alkyle en $C_1$ à $C_5$, alcényle en $C_2$ à $C_5$ ou alcynyle en $C_2$ à $C_5$ linéaire ou ramifié, soit un hétérocycle azoté comprenant de 5 à 10 atomes et éventuellement un ou plusieurs autres hétéroatomes choisis parmi l'azote, l'oxygène et le soufre ;
- soit un groupe NH(C=O)O, auquel cas Y représente un groupe $CR_9R_{10}R_{11}$ dans lequel $R_9$, $R_{10}$ et $R_{11}$ représentent, indépendamment les uns des autres, un atome d'hydrogène ou un groupe alkyle en $C_1$ à $C_5$, alcényle en $C_2$ à $C_5$ et alcynyle en $C_2$ à $C_5$ linéaire ou ramifié.

De manière préférée, ces nouveaux dérivés de la pipérazine de formule particulière (I-a) sont la 1,4-di-(3',4',5'-triméthoxybenzoyl)-2-(1'-pipéridinocarbonyloxyméthyl)-pipérazine, la 1,4-di-(3',4',5'-triméthoxybenzoyl)-2-(1'-pyrrolidinocarbonyloxyméthyl)-pipérazine, la 1,4-di-(3',4'-diméthoxybenzoyl)-2-(N,N-diéthylaminocarbonyloxyméthyl)-pipérazine, la 1,4-di-(3',4',5'-triméthoxybenzoyl)-2-(isopropyloxycarbonylaminométhyl)-pipérazine, la 1,4-di-(3',4',5'-triméthoxybenzoyl)-2-(tertiobutylcarbonylaminométhyl)-pipérazine, la 1-(3',4',5'-triméthoxybenzoyl)-4-(3',4',5'-triméthoxythiobenzoyl)-2-(N,N-di-éthylaminocarbonyloxyméthyl)-pipérazine et la 1,4-di(3',4',5'-triméthoxythiobenzoyl)-2-(N,N-diéthylaminocarbonyloxyméthyl)-pipérazine.

Selon un autre mode de réalisation préféré de ces nouveaux dérivés de la pipérazine, ces derniers répondent à la formule particulière (I-b) :

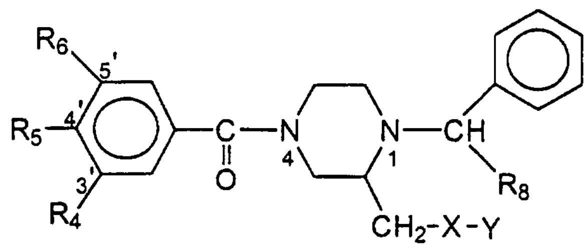

(I-b)

dans laquelle $R_8$ représente un atome d'hydrogène ou un groupe phényle, tandis que $R_4$, $R_5$, $R_6$, X et Y ont la même signification que dans la formule générale (I) définie ci-avant:

De manière avantageuse, dans ces nouveaux dérivés de la pipérazine de formule particulière (I-b), $R_4$, $R_5$ et $R_6$ représentent un groupe méthoxy, X représente un groupe O(C=O), tandis que Y représente un groupe $NR_9R_{10}$ dans lequel $R_9$ et $R_{10}$ représentent tous deux un groupe alkyle en $C_1$ à $C_5$, alcényle en $C_2$ à $C_5$ ou alcynyle en $C_2$ à $C_5$ linéaire ou ramifié, ou bien un hétérocycle azoté comprenant de 5 à 10 atomes et éventuellement un ou plusieurs autres hétéroatomes choisis parmi l'azote, l'oxygène et le soufre.

De manière préférée, les nouveaux dérivés pipéraziniques répondant à la formule particulière (I-b) sont la 1-benzyl-4-(3',4',5'-triméthoxybenzoyl)-2-(N,N-diéthylaminocarbonyloxyméthyl)-pipérazine et la 1-diphénylméthyl-4-(3',4',5'-triméthoxybenzoyl)-2-(N,N-diéthylaminocarbonyloxyméthyl)-pipérazine.

Selon encore un autre mode de réalisation préféré desdits nouveaux dérivés de la pipérazine, ces derniers répondent à la formule particulière (I-c) :

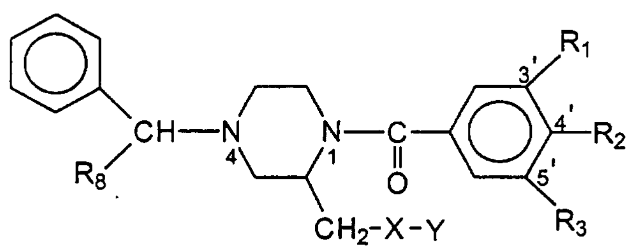

(I-c)

dans laquelle $R_8$ représente un atome d'hydrogène ou un groupe phényle, tandis que $R_1$, $R_2$, $R_3$, X et Y ont la même signification que dans la formule générale (I) définie ci-avant.

De manière avantageuse, dans les nouveaux dérivés de la pipérazine de formule particulière (I-c), $R_1$, $R_2$ et $R_3$ représentent un groupe méthoxy, X représente un groupe O(C=O), tandis que Y représente un groupe $NR_9R_{10}$ dans lequel $R_9$ et $R_{10}$ représentent tous deux un groupe alkyle en $C_1$ à $C_5$, alcényle en $C_2$ à $C_5$ ou alcynyle en $C_2$ à $C_5$ linéaire ou ramifié, ou bien un hétérocycle azoté comprenant de 5 à 10 atomes et éventuellement un ou plusieurs autres hétéroatomes choisis parmi l'azote, l'oxygène et le soufre.

Parmi ces nouveaux dérivés pipéraziniques de formule particulière (I-c), on préfère notamment la 1-(3',4',5'-triméthoxybenzoyl)-4-benzyl-2-(N,N-diéthylaminocarbonyloxyméthyl)-pipérazine.

La présente Invention a aussi pour objet un nouveau dérivé de la pipérazine, lequel dérivé est la 1-(3',4',5'-triméthoxythiobenzoyl)-4-(3',4',5'-triméthoxybenzoyl)-2-(N,N-diéthylaminocarbonyloxyméthyl)-pipérazine, et ses sels pharmaceutiquement acceptables.

La présente Invention a encore pour objet les nouveaux dérivés de la pipérazine tels que définis ci-avant et leurs sels pharmaceutiquement acceptables pour l'utilisation comme médicaments.

La présente Invention a, en outre, pour objet des compositions pharmaceutiques, caractérisées en ce qu'elles comprennent en tant que principe actif, au moins un nouveau dérivé de la pipérazine tel que défini ci-avant, ou l'un de ses sels pharmaceutiquement acceptables, et éventuellement des excipients et additifs pris parmi ceux dont on se sert classiquement en pharmacie. Compte tenu des propriétés inhibitrices présentées par un tel dérivé, à la fois sur la réplication du VIH et sur l'action du PAF, ces compositions pharmaceutiques trouvent notamment application dans la prévention et/ou le traitement de l'infection par le VIH à tous ses stades : séropositivité ou Syndrome d'Immunodéficience Acquise (SIDA).

Outre les dispositions qui précèdent, l'Invention comprend encore d'autres dispositions qui ressortiront du complément de description qui suit, qui se rapporte à des exemples de préparation de dérivés de la pipérazine conformes à l'Invention, et de démonstration de leur activité inhibitrice sur la réplication du VIH et de leur absence de cytotoxicité, ainsi qu'aux dessins annexés dans lesquels :

- la Figure 1 illustre, sous la forme d'une courbe, la relation effet-dose de la 1,4-di-(3',4',5'-triméthoxybenzoyl)-2-(N,N-diéthylaminocarbonyloxyméthyl)-pipérazine sur la réplication du VIH dans des macrophages dérivés des monocytes ;
- la Figure 2 illustre, sous la forme d'un histogramme, l'activité inhibitrice de la 1,4-di-(3',4',5'-triméthoxybenzoyl)-2-(N,N-diéthylaminocarbonyloxyméthyl)-pipérazine sur la réplication du VIH dans différentes populations cellulaires (macrophages dérivés des monocytes, monocytes et cellules mononuclées du sang périphérique) à la dose de 100 μM; et
- la Figure 3 illustre, sous la forme d'un histogramme, la viabilité de macrophages dérivés des monocytes traités par la 1,4-di-(3',4',5'-triméthoxybenzoyl)-2-(N,N-diéthylaminocarbonyloxyméthyl)-pipérazine à la dose de 100 μM et celle de macrophages dérivés des monocytes non traités par ce dérivé.

## I - PREPARATION DE DERIVES DE LA PIPERAZINE

**EXEMPLE 1 : Préparation de la 1,4-di-(3',4',5'-triméthoxybenzoyl)-2-(1'-pyrrolidinocarbonyloxyméthyl)-pipérazine** [dérivé de formule particulière (I-a) dans laquelle A et B = C=O, $R_1$ à $R_6$ = $OCH_3$, X = O(C=O) et Y = pyrrolidinyle]

### 1.1 - Préparation de la 1,4-dibenzyl-2-phénoxycarbonyloxyméthyl pipérazine

Dans un erlenmeyer rodé de 500 ml surmonté d'une ampoule à additionner et placé dans un bain de glace, une solution comprenant 10 g (33,7 mmoles) de 1,4-dibenzyl-2-hydroxyméthylpipérazine (préparée conformément au procédé décrit par JUCKER et RISSI dans *Helv. Chem. Acta*, 1962, 45, 2383-2402) dans 9,5 ml (2,8 équivalents - 94,4 mmoles) de triéthylamine et 95 ml de dichlorométhane ($CH_2Cl_2$) est refroidie. Après addition goutte à goutte de 6,4 g (1,25 équivalents - 42 mmoles) de chlorocarbonate de phényle solubilisés dans 95 ml de $CH_2Cl_2$, le mélange est agité à 0°C pendant une heure, puis à température ambiante pendant 10 heures. En fin de réaction, la solution est lavée une fois par une solution saturée de bicarbonate de sodium ($NaHCO_3$), puis à l'eau jusqu'à l'obtention d'un pH neutre, et elle est séchée sur du sulfate de magnésium ($MgSO_4$). Après filtration sur papier et évaporation du $CH_2Cl_2$, le résidu est chromatographié sur colonne de gel de silice en utilisant du dichlorométhane comme éluant. 8 g de 1,4-dibenzyl-2-phénoxycarbonyloxyméthylpipérazine sont ainsi obtenus sous la forme d'un produit visqueux. Rendement : 57%. Rf= 0,52 (MeOH/$CH_2Cl_2$, 2:98, v/v).

### 1.2 - Préparation de la 1,4-dibenzyl-2-(1'-pyrrolidinocarbonyloxyméthyl)-pipérazine

Dans un erlenmeyer rodé de 100 ml surmonté d'un réfrigérant, 3,12 g (7,5 mmoles) de 1,4-dibenzyl-2-phénoxycarbonyloxyméthylpipérazine sont solubilisés dans 9,4 ml (15 équivalents - 112,8 mmoles) de pyrrolidine. Le mélange est porté à reflux dans un bain d'huile thermostaté pendant 60 heures. Après refroidissement et évaporation de l'amine en excès, le résidu est repris au $CH_2Cl_2$ et lavé à l'eau jusqu'à neutralité. Après séchage de la phase organique ($MgSO_4$), filtration sur papier et évaporation du solvant, le produit est chromatographié sur colonne de gel de silice, l'éluant étant un mélange MeOH/$CH_2Cl_2$ (0,5:99,5, v/v). 1,48 g de 1,4-dibenzyl-2-(1'-pyrrolidinocarbonyloxyméthyl)-pipérazine sont ainsi obtenus sous la forme d'un produit visqueux. Rendement : 50,2%. Rf = 0,38 (MeOH/$CH_2Cl_2$, 5:95, v/v).

**1.3 - Préparation du chlorhydrate de la 2-(1'-pyrrolidinocarbonyloxyméthyl)-pipérazine**

Dans un ballon rodé de 100 ml, 1,48 g (3,7 mmoles) de 1,4-dibenzyl-2-(l'-pyrrolidinocarbonyloxyméthyl)-pipérazine sont solubilisés dans 40 ml d'éthanol absolu et 1 ml d'HCl 12 N. Environ 100 mg de palladium (10%) sur charbon y sont ajoutés. Le mélange est alors placé sous atmosphère d'hydrogène, vigoureusement agité et un léger chauffage est appliqué (40°C). Lorsque le produit initial a disparu (l'évolution de la réaction est suivie par chromatographie sur couche mince éluée par un mélange $MeOH/CH_2Cl_2$ (5:95, v/v), après neutralisation d'un aliquote par quelques grains de $NaHCO_3$), la solution est filtrée sur papier et le catalyseur est rincé plusieurs fois à l'éthanol et à l'eau. Les solvants sont ensuite évaporés sous vide. 1,04 g de chlorhydrate de la 2-(l'-pyrrolidinocarbonyloxyméthyl)-pipérazine sont ainsi obtenus et utilisés tels quels à l'étape suivante. Rendement : 98,3%. Rf = 0,4 ($CHCl_3/MeOH/NH_4OH$, 80:20:2, v/v/v).

**1.4 - Préparation de la 1,4-di-(3',4',5'-triméthoxybenzoyl)-2-(1'-pyrrolidinocarbonyloxyméthyl)-pipérazine**

Dans un erlenmeyer rodé de 100 ml surmonté d'une ampoule à additionner, 1,04 g (3,63 mmoles) de chlorhydrate de la 2-(1'- pyrrolidinocarbonyloxyméthyl)-pipérazine sont solubilisés dans 3 ml (12,4 équivalents - 40,85 mmoles) de triéthylamine et 50 ml de $CH_2Cl_2$. 2,88 g (3,8 équivalents - 12,5 mmoles) de chlorure de 3,4,5-triméthoxybenzoyle solubilisés dans 30 ml de $CH_2Cl_2$ sec sont ajoutés par un goutte à goutte rapide. Après 3 heures d'agitation et l'addition de quelques ml d'éthanol, la solution est lavée deux fois à l'eau, séchée ($MgSO_4$), filtrée sur papier et évaporée sous vide. Le résidu est alors chromatographié sur colonne de gel de silice, l'éluant étant un mélange $MeOH/CH_2Cl_2$ (1:99; v/v). Le produit obtenu est repris dans du $CH_2Cl_2$, lavé par une solution saturée de $NaHCO_3$ pour éliminer les traces d'acide triméthoxybenzoïque et, après évaporation des solvants, cristallisé dans un mélange méthanol/éther (5:95, v/v). 591 mg de cristaux de 1,4-di-(3',4',5'-triméthoxybenzoyl)-2-(1'-pyrrolidinocarbonyloxyméthyl)-pipérazine sont ainsi obtenus après filtration sur fritté, lavage à l'éther et séchage. Rendement : 29%. Rf= 0,43 ($MeOH/CH_2Cl_2$, 5:95, v/v). Point de fusion : 145,1°C.
IR : 1694 (C=O carbamate), 1622 (C=O amide), 1584 (C=C aromatiques) $cm^{-1}$.
RMN $^1H$ (200 MHz, $CDCl_3$, HMDS) $\delta$ ppm : 6,57 (s, 4H, H aromatiques), 5-4,07 (m, 5H, $CH_2OCO$ et $CH_2$ et CH pipéraziniques), 3,8 (s large, 18 H, $CH_3O$), 3,62-2,3 (m, 8H, $CH_2N$), 1,72 (s large, 4H, $CH_2$ pyrrolidine).

**EXEMPLE 2 : <u>Préparation de la 1.4-di-(3',4',5'-triméthoxybenzoyl)-2-(N,N-diéthylaminocarbonyloxyméthyl)-pipérazine</u>** [dérivé de formule particulière (I-a) dans laquelle A et B = C=O, $R_1$ à $R_6$ = $OCH_3$, X = O(C=O) et Y = N$(CH_2CH_3)_2$]

**2.1 - Préparation de la 1,4-dibenzyl-2-(N,N-diéthylaminocarbonyloxyméthyl)-pipérazine**

En suivant un protocole identique à celui utilisé à l'étape 1.1 de l'Exemple 1, puis en traitant, dans les mêmes conditions que celles de l'étape 1.2 de l'Exemple 1, la 1,4-dibenzyl-2-phénoxycarbonyloxyméthylpipérazine par 10 ml de diéthylamine, on obtient à partir de 3g de 1,4-dibenzyl-2-phénoxycarbonyloxyméthylpipérazine, 1,75 g de 1,4-dibenzyl-2-(N,N-diéthylaminocarbonyloxyméthyl)-pipérazine sous la forme d'un produit visqueux. Rendement : 61,7%. Rf= 0,2 (éther/éther de pétrole, 50:50, v/v).

**2.2 - Préparation de la 1,4-di-(3',4',5'-triméthoxybenzoyl)-2-(N,N-diéthylaminocarbonyloxyméthyl)-pipérazine**

En suivant également un protocole identique à celui utilisé à l'étape 1.3 de l'Exemple 1, puis en traitant, dans les mêmes conditions que celles de l'étape 1.4 de l'Exemple 1, 0,5 g (1,73 mmoles) de chlorhydrate de la 2-(N,N-diéthylaminocarbonyloxyméthyl)-pipérazine par 1 g de chlorure d'acide 3,4,5-triméthoxybenzoïque, on obtient 0,83 g de 1,4-di-(3',4',5'-triméthoxybenzoyl)-2-(N,N-diéthylaminocarbonyloxyméthyl)-pipérazine. Rendement : 80%. Point de fusion : 128,6°C. Rf= 0,35 ($MeOH/CH_2Cl_2$, 3:97, v/v).
IR : 1694 (C=O carbamate), 1632 (C=O amide), 1584 (C=C aromatiques) $cm^{-1}$.
RMN $^1H$ (80 MHz, $CDCl_3$, HMDS) $\delta$ ppm : 6,62 (s, 4H, H aromatiques), 5-4,07 (m, 5H, $CH_2OCO$ et $CH_2$ et CH pipéraziniques), 3,8 (s large, 18 H, $CH_3O$), 3,62-2,92 (m, 8H, $CH_2N$), 1,0 (t, 6H, $CH_3$).

**EXEMPLE 3 : Préparation de la 1,4-di-(3',4',5'-triméthoxybenzoyl)-2-(N,N-dipropylaminocarbonyloxyméthyl)-pipérazine** [dérivé de formule particulière (I-a) dans laquelle A et B = C=O, $R_1$ à $R_6$ = $OCH_3$, X = O(C=O) et Y = N $(CH_2CH_2CH_3)_2$]

### 3.1 - Préparation de la 1,4-dibenzyl-2-(N,N-dipropylaminocarbonyloxyméthyl)-pipérazine

En suivant également un protocole identique à celui utilisé à l'étape 1.1 de l'Exemple 1, puis en traitant, dans les mêmes conditions que celles de l'étape 1.2 de l'Exemple 1, 3g (27,2 mmoles) de 1,4-dibenzyl-2-phénoxycarbonyloxyméthyl)-pipérazine par 10 ml de dipropylamine, on obtient 2,28 g de 1,4-dibenzyl-2-(N,N-dipropylaminocarbonyloxyméthyl)-pipérazine sous la forme d'un produit visqueux. Rendement : 75%. Rf= 0,22 (éther/éther de pétrole, 50:50, v/v).

### 3.2 - Préparation de la 1,4-di-(3',4',5'-triméthoxybenzoyl)-2-(N,N-dipropylaminocarbonyloxyméthyl)-pipérazine

En suivant également un protocole identique à celui utilisé à l'étape 1.3 de l'Exemple 1, puis en traitant, dans les mêmes conditions que celles de l'étape 1.4 de l'Exemple 1, 0,8 g de chlorhydrate de la 2-(N,N-propylaminocarbonyloxyméthyl)-pipérazine par 1,47 g de chlorure d'acide 3,4,5-triméthoxybenzoïque, on obtient 1,24 g de 1,4-di-(3',4',5'-triméthoxybenzoyl)-2-(N,N-dipropylaminocarbonyloxyméthyl)-pipérazine. Rendement : 78%. Point de fusion : 109,3°C. Rf = 0,36 ($MeOH/CH_2Cl_2$, 3:97, v/v).
IR : 1700 (C=O carbamate), 1640 (C=O amide), 1590 (C=C aromatiques) $cm^{-1}$.
RMN $^1H$ (80 MHz, $CDCl_3$, HMDS) $\delta$ ppm : 6,62 (s, 4H, H aromatiques), 5-4,07 (m, 5H, $CH_2OCO$ et $CH_2$ et CH pipéraziniques), 3,8 (s large, 18 H, $CH_3O$), 3,62-2,92 (m, 8H, $CH_2N$), 1,3 (m, 4H, $CH_2$), 0,8 (t, 6H, $CH_3$).

**EXEMPLE 4 : Préparation de la 1,4-di-(3',4',5'-triméthoxybenzoyl)-2-(1'-pipéridinocarbonyloxyméthyl)-pipérazine** [dérivé de formule particulière (I-a) dans laquelle A et B = C=O, $R_1$ à $R_6$ = $OCH_3$, X = O(C=O) et Y = pipéridinyle]

### 4.1 - Préparation de la 1,4-dibenzyl-2-(1'-pipéridinocarbonyloxyméthyl)-pipérazine

En suivant également un protocole identique à celui utilisé à l'étape 1.1 de l'Exemple 1, puis en traitant, dans les mêmes conditions que celles de l'étape 1.2 de l'Exemple 1, 1 g de 1,4-dibenzyl-2-phénoxycarbonyloxyméthylpipérazine par 10 ml de pipéridine, on obtient 0,68g de 1,4-dibenzyl-2-(1'-pipéridinocarbonyloxyméthyl)-pipérazine sous la forme d'un produit visqueux. Rendement : 66,5%. Rf= 0,09 (éther/éther de pétrole, 70:30, v/v).

### 4.2 - Préparation de la 1,4-di-(3',4',5'-triméthoxybenzoyl)-2-(1'-pipéridinocarbonyloxyméthyl)-pipérazine

En suivant également un protocole identique à celui utilisé à l'étape 1.3 de l'Exemple 1, puis en traitant, dans les mêmes conditions que celles de l'étape 1.4 de l'Exemple 1, 0,5 g de chlorhydrate de la 2-(1'-pipéridinocarbonyloxyméthyl)-pipérazine par 1,1 g de chlorure d'acide de 3,4,5-triméthoxybenzoïque, on obtient 0,8 g de 1,4-di-(3',4',5'-triméthoxybenzoyl)-2-(1'-pipéridinocarbonyloxyméthyl)-pipérazine. Rendement : 56%. Point de fusion : 146,7°C.
IR : 1694 (C=O carbamate), 1622 (C=O amide), 1584 (C=C aromatiques) $cm^{-1}$.
RMN $^1H$ (200 MHz, $CDCl_3$, HMDS) $\delta$ ppm : 6,56 (s, 4H, H aromatiques), 5,3-4 (m, 5H, $CH_2OCO$ et $CH_2$ et CH pipéraziniques), 3,8 (s large, 18 H, $CH_3O$), 3,60-2,70 (m, 8H, $CH_2N$), 1,43 (m, 6H, $CH_2$ pipéridiniques).

**EXEMPLE 5 : Préparation de la 1,4-di-(3',4'-diméthoxybenzoyl)-2-(N,N-diéthylaminocarbonyloxyméthyl)-pipérazine** [dérivé de formule particulière (I-a) dans laquelle A et B = C=O, $R_1$, $R_2$, $R_4$ et $R_5$ = $OCH_3$, $R_3$ et $R_6$ = H, X = O (C=O) et Y = $N(CH_2CH_3)_2$]

En suivant également un protocole identique à celui utilisé à l'étape 1.3 de l'Exemple 1, puis en traitant, dans les mêmes conditions que celles de l'étape 1.4 de l'Exemple 1, 3,36 g (11,6 mmoles) de chlorhydrate de la 2-(N,N-diéthylaminocarbonyloxyméthyl)-pipérazine par 5,8 g (2,5 équivalents - 29 mmoles) de chlorure de 3,4-diméthoxybenzoyle, on obtient après 4 heures de réaction et une chromatographie sur colonne de gel de silice éluée par un mélange $MeOH/CH_2Cl_2$ (1:99, v/v), 3,35 g de 1,4-di-(3',4'-diméthoxybenzoyl)-2-(N,N-diéthylaminocarbonyloxyméthyl)-pipérazine. Rendement : 53,2%. Point de fusion : 103,9°C. Rf = 0,49 ($MeOH/CH_2Cl_2$, 5:95, v/v).
IR : 1694 (C=O carbamate), 1622 (C=O amide), 1584 (C=C aromatiques) $cm^{-1}$.
RMN $^1H$ (200 MHz, $CDCl_3$, HMDS) $\delta$ ppm : 6,94 (s, 2H, H aromatiques), 6,85 (dd, 4H, H aromatiques), 5,1-3,9 (m, 5H, $CH_2OCO$ et $CH_2$ et CH pipéraziniques), 3,8 (s large, 12 H, $CH_3O$), 3,9-2,7 (m, 8H, $CH_2N$), 1,3-0,7 (m, 6H, $CH_3$).

**EXEMPLE 6 : Préparation de la 1,4-di-(3',4',5'-triméthoxybenzoyl)2-(isopropyloxycarbonylaminométhyl)-pipérazine** [dérivé de formule particulière (I-a) dans laquelle A et B = C=O, $R_1$ à $R_6$ = $OCH_3$, X = NH(C=O)O et Y = $CH(CH_3)_2$]

**6.1 - Préparation de la 1,4-dibenzyl-2-cyanopipérazine**

Dans un erlenmeyer rodé équipé d'un réfrigérant, 25 g (117 mmoles) de 2,3-dibromopropionitrile sont solubilisés dans 300 ml de benzène et 100 ml de triéthylamine et chauffés à 40°C. 28,17 g (117 mmoles) de N,N'-dibenzyléthylènediamine dissous dans 100 ml de benzène et chauffés à 80°C y sont ajoutés goutte à goutte et l'ensemble est agité pendant 3 heures à 80°C. Après refroidissement, le bromhydrate de triéthylammonium est filtré. Les solvants (benzène et triéthylamine en excès) sont évaporés et le résidu repris à l'éther est lavé par une solution saturée de $NaHCO_3$, puis à l'eau jusqu'à pH neutre. La phase organique est séchée ($MgSO_4$), filtrée et évaporée. On obtient un composé visqueux qui cristallise dans un mélange éther/hexane (60:40, v/v) sous la forme de 28 g de cristaux blancs de 1,4-dibenzyl-2-cyanopipérazine. Rendement : 82%. Point de fusion : 61,7°C. Rf= 0,8 (MeOH/$CH_2Cl_2$, 5:95, v/v).

**6.2 - Préparation de la 1,4-dibenzyl-2-aminométhylpipérazine**

Dans un erlenmeyer rodé d'un litre, 28 g (95 mmoles) de 1,4-dibenzyl-2-cyanopipérazine sont dissous dans 300 ml d'éther et ajoutés goutte à goutte à une suspension agitée de 10,96 g (3 équivalents - 284 mmoles) de $LiAlH_4$ dans 50 ml d'éther. Le mélange est maintenu sous agitation 12 heures à température ambiante. A la fin de la réaction, il est hydrolysé par addition goutte à goutte d'une solution de soude à 20%. Le complexe solide d'hydroxyde d'aluminium qui se forme est décanté et rincé à l'éther. La phase éthérée est lavée à l'eau, séchée ($MgSO_4$), filtrée et évaporée et le résidu obtenu est purifié par chromatographie sur colonne de gel de silice utilisant comme éluant un mélange MeOH/$CH_2Cl_2$ (1:99, v/v). On récupère ainsi 22 g de 1,4-dibenzyl-2-aminométhylpipérazine sous la forme d'un produit visqueux. Rendement : 77,5%. Rf= 0,2 ($CH_2Cl_2$/MeOH, 80:20, v/v).

**6.3 - Préparation de la 1,4-dibenzyl-2-isopropyloxycarbonylaminométhylpipérazine et de son chlorhydrate**

Dans un erlenmeyer rodé, 22 g (75 mmoles) de 1,4-dibenzyl-2-aminométhylpipérazine dissous dans 300 ml de toluène sont mélangés à 100 ml de triéthylamine. 97 ml (0,1 mole) de chloroformate d'isopropyle prélevés à l'aide d'une seringue sont mis en solution dans 50 ml de toluène, puis ajoutés goutte à goutte à la solution précédente. A la fin de l'addition, le mélange est agité pendant 2 heures à température ambiante. La solution est diluée à l'éther, lavée avec une solution saturée de $NaHCO_3$, puis à l'eau jusqu'à l'obtention d'un pH neutre. La phase organique est séchée ($MgSO_4$), filtrée, évaporée et donne 28,3 g de 1,4-dibenzyl-2-isopropyloxycarbonylaminométhylpipérazine sous la forme d'un composé visqueux. Rendement : 99,6%. Rf= 0,4 ($CH_2Cl_2$/MeOH, 95:5, v/v).

La 1,4-dibenzyl-2-isopropyloxycarbonylaminométhylpipérazine est chlorhydratée par dissolution dans l'éthanol et saturation avec de l'HCl gazeux. Après évaporation de l'éthanol, on obtient 32,3 g de chlorhydrate de 1,4-dibenzyl-2-isopropyloxycarbonylaminométhylpipérazine qui sont utilisés tels quels dans l'étape suivante.

**6.4 - Préparation du chlorhydrate de la 2-isopropyloxycarbonylaminométhylpipérazine**

32,3 g (71 mmoles) de chlorhydrate de la 1,4-dibenzyl-2-isopropyloxycarbonylaminométhylpipérazine sont dissous dans 100 ml d'acide acétique et 200 mg de palladium (10%) sur charbon sont ajoutés. Grâce à un appareil de PARR, la suspension est agitée et chauffée à 60°C sous pression d'hydrogène (2,7 bars) pendant une nuit. Le catalyseur est filtré et le solvant évaporé pour donner un composé qui est cristallisé dans un mélange éthanol/éther (5:95, v/v) pour donner 17 g de chlorhydrate de 2-isopropyloxycarbonylaminométhylpipérazine sous la forme de cristaux blancs. Rendement : 87%. Point de fusion : 237,1°C. Rf = 0,6 ($CHCl_3$/MeOH/$NH_4OH$, 80:20:2, v/v/v).

**6.5 - Préparation de la 1,4-di-(3',4',5'-triméthoxybenzoyl)-2-(isopropyloxycarbonylaminométhyl)-pipérazine**

Dans un erlenmeyer d'un litre, 17 g (60 mmoles) de chlorhydrate de 2-isopropyloxycarbonylaminométhylpipérazine sont solubilisés dans 300 ml de dichlorométhane et 50 ml de triéthylamine. 31,5 g (114 mmoles) de chlorure de 3,4,5-triméthoxybenzoyle solubilisés dans 150 ml de $CH_2Cl_2$ sont ajoutés goutte à goutte à l'aide d'une ampoule à additionner. Le mélange est agité pendant 3 heures. La solution est ensuite lavée avec une solution saturée de $NaHCO_3$, puis à l'eau jusqu'à l'obtention d'un pH neutre. La phase organique est séchée ($MgSO_4$), filtrée, évaporée sous pression réduite et le résidu purifié par chromatographie sur colonne de gel de silice utilisant un mélange MeOH/$CH_2Cl_2$ (1:99, v/v) comme éluant. On obtient ainsi 26,3 g de 1,4-di-(3',4',5'-triméthoxybenzoyl)-2-(isopropyloxycarbonylaminométhyl)-pipérazine après recristallisation dans un mélange acétone/éther de pétrole (70:30, v/v). Rendement : 72%. Point de fusion : 140,2°C. Rf= 0,3 ($CH_2Cl_2$/MeOH, 95:5, v/v).

IR : 3368 (N-H), 1717 (C=O carbamate), 1622 (C=O amide), 1584 (C=C aromatiques) $cm^{-1}$.
RMN $^1H$ : (200 MHz, $CDCl_3$, HMDS) δ ppm : 6,59 (s, 4H, H aromatiques), 5,15 (massif, 1H, NH), 4,85 (quintuplet, 1H, CH pipérazinique), 4,5-4 (m, 3H, CHOCO et $CH_2NCOO$), 3,8 (s large, 18H, $CH_3O$), 3,6-2,9 (m, 6H, $CH_2N$), 1,15 (m, 6H, $CH_3$).

**EXEMPLE 7 : Préparation de la 1,4-di-(3',4',5'-triméthoxybenzoyl)-2-(tertiobutylcarbonylaminométhyl)-pipérazine** [dérivé de formule particulière (I-a) dans laquelle A et B = C=O, $R_1$ à $R_6$ = $OCH_3$, X = NH(C=O) et Y = $C(CH_3)_3$]

**7.1 - Préparation de la 1,4-dibenzyl-2-tertiobutylcarbonylaminométhylpipérazine**

3,36 g (13,1 mmoles) de 1,4-dibenzyl-2-aminométhylpipérazine préparés en suivant un protocole identique à celui utilisé aux étapes 6.1 et 6.2 de l'Exemple 6 sont dissous dans 5,5 ml (3 équivalents - 39,3 mmoles) de triéthylamine et 100 ml de dichlorométhane et traités par un ajout, par un goutte à goutte rapide, de 2,63 ml (1,5 équivalents - 19,6 mmoles) de chlorure de 2,2-diméthylpropanoyle dissous dans 30 ml de $CH_2Cl_2$. La solution est chauffée à reflux pendant 4 heures. Quelques ml d'éthanol sont ensuite ajoutés à la solution qui est alors lavée deux fois à l'eau, une fois avec une solution saturée de $NaHCO_3$, puis à l'eau jusqu'à l'obtention d'un pH neutre. Après séchage de la phase organique ($MgSO_4$), filtration sur papier et évaporation des solvants, le résidu est chromatographié sur colonne de gel de silice, l'éluant étant un mélange $MeOH/CH_2Cl_2$ (0,5:99,5, v/v). 2,44 g de 1,4-dibenzyl-2-tertiobutylcarbonylaminométhylpipérazine sont ainsi obtenus sous la forme d'un produit visqueux. Rendement : 49%. Rf= 0,36 ($CH_2Cl_2$/MeOH, 95:5, v/v).

**7.2 - Préparation du chlorhydrate de la 2-tertiobutylcarbonylaminométhylpipérazine**

Dans un ballon rodé de 100 ml, 2,44 g (6,43 mmoles) de 1,4-dibenzyl-2-tertiobutylcarbonylaminométhylpipérazine sont solubilisés dans 50 ml d'éthanol et 1 ml dTHCl 12 N. Environ 100 mg de palladium (10%) sur charbon y sont ajoutés. Le mélange est alors placé sous atmosphère d'hydrogène, vigoureusement agité et légèrement chauffé (40°C) pendant 3 heures. La suspension est filtrée sur papier et le catalyseur rincé plusieurs fois à l'éthanol et à l'eau. Les solvants sont évaporés et l'on obtient 1,2 g de chlorhydrate de la 2-tertiobutylcarbonylaminométhylpipérazine. Rendement : 68,6%. Point de fusion : 278,6°C. Rf=0,13 ($CHCl_3/MeOH/NH_4OH$, 80:20, v/v/v).

**7.3 - Préparation de la 1,4-di-(3',4',5'-triméthoxybenzoyl)-2-tertiobutylcarbonylaminométhylpipérazine**

En suivant le protocole décrit dans l'étape 6.5 de l'Exemple 6, mais à partir de 1,2 g (4,4 mmoles) de 2-tertiobutylcarbonylaminométhylpipérazine et 2,33 g (2,3 équivalents - 10,1 mmoles) de chlorure de 3,4,5-triméthoxybenzoyle, on obtient 900 mg de 1,4-di-(3',4',5'-triméthoxybenzoyl)-2-tertiobutylcarbonylaminométhylpipérazine, après 24 heures de réaction à température ambiante et une chromatographie sur colonne de gel de silice éluée par un mélange $MeOH/CH_2Cl_2$ (2:98, v/v). Rendement : 34,6%. Rf = 0,29 ($CH_2Cl_2$/MeOH, 95:5, v/v). Point de fusion : 146,5°C.

**EXEMPLE 8 : Préparation du chlorhydrate de la 1-benzyl-4-(3',4',5'-triméthoxybenzoyl)-2-(N,N-diéthylaminocarbonyloxyméthyl)-pipérazine** [dérivé de formule particulière (I-b) dans laquelle $R_4$ à $R_6$ = $OCH_3$, $R_8$ = H, X = O (C=O) et Y = $N(CH_2CH_3)_2$]

**8.1 - Préparation de la 4-(3',4',5'-triméthoxybenzoyl)-2-(N,N-diéthylaminocarbonyloxyméthyl)-pipérazine**

Dans un erlenmeyer rodé d'un litre surmonté d'une ampoule à additionner et plongé dans un bain de glace, 19,86 g (86 mmoles) de dichlorhydrate de 2-(N,N-diéthylaminocarbonyloxyméthyl)-pipérazine préparé conformément à l'étape 2.2 de l'Exemple 2 sont dissous dans 400 ml de $CH_2Cl_2$ et 42 ml (3,5 équivalents) de triéthylamine. A la solution ainsi obtenue, on ajoute goutte à goutte une solution contenant 19,83 g (86 mmoles) de chlorure de 3,4,5-triméthoxybenzoyle dans 200 ml de $CH_2Cl_2$. La réaction est suivie en chromatographie sur couche mince en utilisant un mélange $CH_2Cl_2/MeOH/NK_4OH$ (80:20:0,5, v/v/v) comme éluant, jusqu'à l'élimination de l'amine de départ. La solution est alors lavée avec une solution saturée de $NaHCO_3$, puis à l'eau jusqu'à l'obtention d'un pH neutre. La phase organique est alors séchée ($MgSO_4$), filtrée, évaporée, et le résidu obtenu est chromatographié sur colonne de gel de silice avec un mélange $MeOH/CH_2Cl_2$ (1:99, v/v) comme éluant. On obtient ainsi 18,75 g de 4-(3',4',5'-triméthoxybenzoyl)-2-(N,N-diéthylaminocarbonyloxyméthyl)-pipérazine sous la forme d'une huile visqueuse. Rendement : 53,33%.

**8.2 - Préparation du chlorhydrate de la 1-benzyl-4-(3',4',5'-triméthoxybenzoyl)-2-(N,N-diéthylaminocarbonyloxyméthyl)-pipérazine**

Dans un erlenmeyer rodé de 500 ml, on introduit 20,8 g (51 mmoles) de 4-(3',4',5'-triméthoxybenzoyl)-2-(N,N-diéthylaminocarbonyloxyméthyl)-pipérazine, 7,74 g (61 mmoles) de chlorure de benzyle, 200 ml d'acétonitrile, 10 g de carbonate de potassium ($K_2CO_3$) et 1 g d'iodure de potassium (KI). L'ensemble est chauffé à reflux pendant 2 heures. La suspension obtenue est filtrée sur fritté et le solide ainsi récupéré est lavé à l'acétone. Le filtrat est évaporé, puis il est repris au $CH_2Cl_2$ et lavé à l'eau. La phase organique est séchée ($MgSO_4$), filtrée, évaporée et le résidu obtenu, purifié sur colonne de gel de silice avec une élution par un mélange $MeOH/CH_2Cl_2$ (2:98, v/v), conduit à l'obtention d'un produit qui est redissout dans de l'éthanol absolu et chlorhydraté par barbotage d'un courant d'HCl gazeux. Après évaporation de l'éthanol, on cristallise le chlorhydrate ainsi formé dans un mélange acétone/éther (60:40, v/v), ce qui conduit à l'obtention de 26,46 g de cristaux de chlorhydrate de la 1-benzyl-4-(3',4',5'-triméthoxybenzoyl)-2-(N,N-diéthylaminocarbonyloxyméthyl)-pipérazine. Rendement : 96,9%. Point de fusion : 134°C.
IR : 1690 (C=O carbamate), 1631 (C=O amide), 1586 (C=C aromatiques) $cm^{-1}$.
RMN $^1H$ (200 MHz, $CDCl_3$, HMDS) δ ppm : 13,38 (massif, 1H, HCl), 7,58-7,39 (2 s larges, 5H, H benzyliques aromatiques), 6,55 (s, 2H, H aromatiques), 5,15-3,9 (m, 7H, $CH_2OCO$, $CH_2$ benzylique et $CH_2$ et CH pipéraziniques), 3,78 (s large, 9 H, $CH_3O$), 3,6-2,1 (m, 8H, $CH_2N$), 1,05 (t, 6H, $CH_3$).

**EXEMPLE 9 : Préparation du chlorhydrate de la 1-diphénylméthyl-4-(3',4',5'-triméthoxybenzoyl)-2-(N,N-diéthylaminocarbonyloxyméthyl)-pipérazine** [dérivé de formule particulière (I-b) dans laquelle $R_4$ à $R_6$ = $OCH_3$, $R_8$ = phényle, X = O(C=O) et Y = $N(CH_2CH_3)_2$]

Dans un erlenmeyer rodé de 50 ml surmonté d'un réfrigérant et placé dans un bain d'huile, 300 mg (0,67 mmoles) de 4-(3',4',5'-triméthoxybenzoyl)-2-(N,N-diéthylaminocarbonyloxyméthyl)-pipérazine préparée conformément à l'étape 8.1 de l'Exemple 8, 320 mg (2 équivalents) de bromure de diphénylméthyle, 60 mg (0,5 équivalent) d'iodure de potassium et 560 mg (6 équivalents) de carbonate de potassium sont agités à reflux dans 10 ml d'acétonitrile pendant 60 heures. La suspension est ensuite filtrée sur papier et les sels recueillis sont rincés plusieurs fois à l'acétonitrile. Le filtrat est évaporé et le résidu est repris au $CH_2Cl_2$ et lavé successivement avec une solution saturée de $NaHCO_3$ et à l'eau jusqu'à l'obtention d'un pH neutre. Une chromatographie sur colonne de gel de silice avec une élution par un mélange $MeOH/CH_2Cl_2$ (0,5:99,5, v/v) permet d'isoler un produit qui est redissous dans de l'éthanol absolu et chlorhydraté par barbotage d'un courant d'HCl gazeux. L'addition d'éther fait précipiter 90 mg de chlorhydrate de la 1-diphénylméthyl-4-(3',4',5'-triméthoxybenzoyl)-2-(N,N-diéthylaminocarbonyloxyméthyl)-pipérazine. Rendement : 22%. Point de fusion : 189,5°C.
IR : 3349 ($N^+$-H), 1694 (C=O carbamate), 1623 (C=O amide), 1580 (C=C aromatiques) $cm^{-1}$.
RMN $^1H$ (200 MHz, $CDCl_3$, HMDS) δ ppm : 7,9 (dd, 4H, H *ortho* des phényle), 7,3 (m, 6H, H aromatiques), 6,55 (s, 2H, H aromatiques), 5,06 (s, 1H, CH du diphénylméthyle), 4,6 et 3,2 (2m, 13H, $CH_2OCO$, $CH_2$ et CH pipéraziniques), 3,78 (s large, 9 H, $CH_3O$), 0,8 (m, 6H, $CH_3$).

**EXEMPLE 10 : Préparation du chlorhydrate de la 1-(3',4',5'-triméthoxybenzoyl)-4-benzyl-2-(N,N-diéthylaminocarbonyloxyméthyl)-pipérazine** [dérivé de formule particulière (I-c) dans laquelle $R_1$ à $R_3$ = $OCH_3$, $R_8$ = H, X = O(C=O) et Y = $N(CH_2CH_3)_2$]

**10.1 - Préparation de la 4-triphénylméthyl-2-(N,N-diéthylaminocarbonyloxyméthyl)-pipérazine**

Dans un erlenmeyer rodé de 1 litre, une solution comprenant 24,68 g (86 mmoles) de chlorhydrate de 2-(N,N-diéthylaminocarbonyloxyméthyl)-pipérazine préparé en suivant un protocole identique à l'étape 1.3 de l'Exemple 1 et dissous dans 400 ml de $CH_2Cl_2$ et 42 ml (3,5 équivalents) de triéthylamine est refroidie à -10°C. A cette solution ainsi refroidie, on ajoute goutte à goutte, sur une durée d'environ 2 heures, une solution comprenant 233 g (86 mmoles) de chlorure de triphénylméthyle dans 200 ml de $CH_2Cl_2$. Après agitation pendant 3 heures à la même température, le mélange est lavé avec une solution saturée de $NaHCO_3$, puis à l'eau jusqu'à l'obtention d'un pH neutre. Après séchage ($MgSO_4$), filtration et évaporation de la phase organique, 35,9 g de 4-triphénylméthyl-2-(N,N-diéthylaminocarbonyloxyméthyl)-pipérazine sont obtenus sous la forme d'un produit visqueux. Rendement : 91,6%.

**10.2 - Préparation de la 1-(3',4',5'-triméthoxybenzoyl)-4-triphénylméthyl-2-(N,N-diéthylaminocarbonyloxyméthyl)-pipérazine**

Dans un erlenmeyer rodé de 500 ml surmonté d'une ampoule à additionner et placé dans un bain de glace, 24,16 g (53 mmoles) de 4-triphénylméthyl-2-(N,N-diéthylaminocarbonyloxyméthyl)-pipérazine sont solubilisés dans

15 ml (2 équivalents) de triéthylamine et 100 ml de $CH_2Cl_2$. A la solution ainsi obtenue, on ajoute goutte à goutte une solution comprenant 14,6 g (1,2 équivalents) de chlorure de 3,4,5-triméthoxybenzoyle dans 25 ml de $CH_2Cl_2$. La réaction est suivie en chromatographie sur couche mince en utilisant de l'éther comme éluant et, lorsque la réaction est terminée, le mélange est lavé avec une solution saturée de $NaHCO_3$, puis à l'eau jusqu'à l'obtention d'un pH neutre. Le résidu obtenu après séchage ($MgSO_4$), filtration et évaporation du solvant, consiste en 32,32 g de 1-(3',4',5'-triméthoxybenzoyl)-4-triphénylméthyl-2-(N,N-diéthylaminocarbonyloxyméthyl)-pipérazine qui sont utilisés tels quels dans l'étape suivante. Rendement : 93,4%.

**10.3 - Préparation de la 1-(3',4',5'-triméthoxybenzoyl)-2-(N,N-diéthylaminocarbonyloxyméthyl)-pipérazine**

Dans un ballon rodé de 500 ml, 26,77 g (41 mmoles) de 1-(3',4',5'-triméthoxybenzayl)-4-triphénylméthyl-2-(N,N-diéthylaminocarbonyloxyméthyl)-pipérazine sont dissous dans 200 ml de méthanol et chlorhydratés par barbotage d'un courant d'HCl gazeux à froid, puis sous agitation à température ambiante et ce, jusqu'à disparition totale du produit de départ. Après évaporation du méthanol, le résidu est repris au $CH_2Cl_2$ et lavé avec une solution saturée de $NaHCO_3$, puis à l'eau jusqu'à l'obtention d'un pH neutre. Après séchage ($MgSO_4$), filtration et évaporation de la phase organique, une chromatographie sur colonne de gel de silice avec une élution par du $CH_2Cl_2$ permet d'obtenir 12,54 g de 1-(3'-4'-5'-triméthoxybenzoyl)-2-(N,N-diéthylaminocarbonyloxyméthyl)-pipérazine sous la forme d'un produit visqueux. Rendement : 74,8%.

**10.4 - Préparation du chlorhydrate de la 1-(3',4',5'-triméthoxybenzoyl)-4-benzyl-2-(N,N-diéthylaminocarbonyloxyméthyl)-pipérazine**

En suivant un protocole identique à celui utilisé à l'étape 8.2 de l'exemple 8, on obtient à partir de 10,63 g (26 mmoles) de 1-(3',4',5'-triméthoxybenzoyl)-2-(N,N-diéthylaminocarbonyloxyméthyl)-pipérazine, 11,55 g de chlorhydrate de la 1-(3',4',5'-triméthoxybenzoyl)-4-benzyl-2-(N,N-diéthylaminocarbonyloxyméthyl)-pipérazine. Rendement : 83%. Point de fusion : 180,6°C.

Les caractéristiques spectrales infra-rouges (IR) et en résonance magnétique nucléaire (RMN) de ce composé sont identiques à celles du chlorhydrate de la 1-benzyl-4-(3',4',5'-triméthoxybenzoyl)-2-(N,N-diéthylaminocarbonyloxyméthyl)-pipérazine dont il représente l'isomère.

**EXEMPLE 11 : Préparation de la 1-(3',4',5'-triméthoxybenzoyl)-4-(3',4',5'-triméthoxythiobenzoyl)-2-(N,N-diéthylaminocarbonyloxyméthyl)-pipérazine** [dérivé de formule particulière (I-a) dans laquelle A = C=O, B = C=S, $R_1$ à $R_6$ = $OCH_3$, X = O(C=O) et Y = $N(CH_2CH_3)_2$]

**11.1 - Préparation de la 4-(3',4',5'-triméthoxythiobenzoyl)-2-(N,N-diéthylaminocarbonyloxyméthyl)-pipérazine**

1,7 g (3,66 mmoles) de 4-(3',4',5'-triméthoxybenzoyl)-2-(N,N-diéthylaminocarbonyloxyméthyl)-pipérazine préparée en suivant le protocole décrit à l'étape 8.1 de l'Exemple 8 sont dissous dans 34 ml de THF et refroidis dans un bain de glace. 1,7 g (1 équivalent) de réactif de LAWESSON y sont alors ajoutés par portions. La solution est agitée à 0°C pendant 30 minutes puis à température ambiante pendant une nuit. Le solvant est ensuite éliminé sous vide et le résidu rapidement purifié sur colonne de gel de silice (éluants : $CH_2Cl_2$ puis 1% de MeOH dans $CH_2Cl_2$) pour donner 1,1 g de 4-(3',4',5'-triméthoxythiobenzoyl)-2-(N,N-diéthylaminocarbonyloxyméthyl)-pipérazine sous forme d'un produit visqueux jaune. Rendement : 62,4%.
IR : 3314 (NH), 2967 (CH), 2836 ($CH_3O$), 1696 (C=O carbamate), 1582 (C=C aromatiques) $cm^{-1}$.

**11.2 - Préparation de la 1-(3',4',5'-triméthoxybenzoyl)-4-(3',4',5'-triméthoxythiobenzoyl)-2-(N,N-diéthylaminocarbonyloxyméthyl)-pipérazine**

Dans un ballon de 25 ml surmonté d'une garde à $CaCl_2$, 250 mg (0,6 mmoles) de 4-(3',4',5'-triméthoxybenzoyl)-2-(N,N-diéthylaminocarbonyloxyméthyl)-pipérazine, 164 µl (2 équivalents) de $Et_3N$ et 200 mg (1,5 équivalent) de chlorure de 3,4,5-triméthoxybenzoyle sont dissous dans 10 ml de $CH_2Cl_2$ et agités à température ambiante pendant une nuit. La solution est alors lavée par de l'eau (2 x 10 ml), séchée, filtrée et évaporée. Le résidu est ensuite purifié sur colonne de gel de silice (élution par 1% de MeOH dans $CH_2Cl_2$) et cristallisé dans un mélange MeOH/diéthyléther/hexane pour donner 150 mg de 1-(3',4',5'-triméthoxybenzoyl)-4-(3',4',5'-triméthoxythiobenzoyl)-2-(N,N-diéthylaminocarbonyloxyméthyl)-pipérazine. Rendement : 41%. Rf= 0,53 (MeOH/$CH_2CH_2$, 5:95, v/v). Point de fusion : 105,8°C.
IR: 2967 (CH), 2839 ($CH_3O$), 1691 (C=O amide), 1584, 1506 (C=C aromatiques) $cm^{-1}$.
RMN $^1H$ (200 MHz, $CDCl_3$, HMDS) δ ppm : 6,58 (s, 2H, CH pipéraziniques), 4,5-3,9 (m, 3H, CH pipérazinique, $CH_2OCO$), 3,79 (s, 18H, $CH_3O$), 3,6-2,6 (massif, 8H, CH pipéraziniques, $CH_2NCO$), 1,03 (m, 6H, $CH_3$).

**EXEMPLE 12 : Préparation de la 1,4-di(3',4',5'-triméthoxybenzoyl)-2-(N,N-diéthylaminocarbonyloxyméthyl)-pipérazine** [dérivé de formule particulière (I-a) dans laquelle A et B = C=S, $R_1$ à $R_6$ = $OCH_3$, X = O(C=O) et Y = N$(CH_2CH_3)_2$]

500 mg (0,83 mmoles) de 1,4-di(3',4',5'-triméthoxybenzoyl)-2-(N,N-diéthylaminocarbonyloxyméthyl)-pipérazine préparée selon l'Exemple 2, sont dissous dans 10 ml de THF et refroidis dans un bain de glace. 670 mg (2 équivalents) de réactif de LAWESSON y sont ajoutés par portions. La solution est maintenue à 0°C pendant 30 minutes, puis 20 heures à température ambiante, et chauffée une heure à reflux. Après refroidissement et évaporation du solvant, le résidu est purifié sur colonne de gel de silice (élution par $MeOH/CH_2Cl_2$, 1:99, v/v). 460 mg de 1,4-di(3',4',5'-triméthoxybenzoyl)-2-(N,N-diéthylaminocarbonyloxyméthyl)-pipérazine sont ainsi obtenus sous forme de cristaux jaunes. Rendement: 87,3%. Point de fusion : 95,1°C.
IR : 2936 (CH), 2833 ($CH_3O$), 1700 (C=O carbamate), 1582, 1507 (C=C aromatiques) $cm^{-1}$.
RMN $^1H$ (200 MHz, $CDCl_3$, HMDS) δ ppm : 7,2 (massif, 4H, H aromatiques), 5,15 et 4,9 (2 massifs, 2H, CH pipéraziniques), 4,4-3,9 (massif, 3H, CH pipérazinique, $CH_2OCO$), 3,79 (s, 18H, $CH_3O$), 3,6-2,1 (m, 8H, $CH_2$ pipéraziniques, $CH_2NCO$), 0,9 (m, 6H, $CH_3$).

**EXEMPLE 13 : Préparation de la 1-(3',4',5'-triméthoxythiobenzoyl)-4-(3',4',5'-triméthoxybenzoyl)-2-(N,N-diéthylaminocarbonyloxyméthyl)-pipérazine** [dérivé de formule particulière (I-a) dans laquelle A = C=S, B = C=O, $R_1$ à $R_6$ = $OCH_3$, X = O(C=O) et Y = N$(CH_2CH_3)_2$]

**13.1 - Préparation de la 1-(3',4',5'-triméthoxythiobenzoyl)-2-(N,N-diéthylaminocarbonyloxyméthyl)-pipérazine**

1,4 g (3,44 mmoles) de 1-(3',4',5'-triméthoxybenzoyl)-2-(N,N-diéthylaminocarbonyloxyméthyl)-pipérazine préparée en suivant le protocole décrit aux étapes 10.1 à 10.3 de l'Exemple 10 sont dissous dans 30 ml de THF et refroidi dans un bain de glace. On y ajoute progressivement 1,4 g (1 équivalent) de réactif de LAWESSON. La solution est maintenue au froid pendant une heure, puis à température ambiante pendant une nuit. Après l'élimination du solvant sous vide et une rapide purification sur gel de silice (élution : $MeOH/CH_2CL_2$, 1:99, v/v), 1,2 g de 1-(3',4',5'-triméthoxythiobenzoyl)-2-(N,N-diéthylaminocarbonyloxyméthyl)-pipérazine sont obtenus. Rendement : 82%.
IR :1690 (C=O carbamate), 1592 (C=C aromatiques) $cm^{-1}$.

**13.2 - Préparation de la 1-(3',4',5'-triméthoxythiobenzoyl)-4-(3',4',5'-triméthoxybenzoyl)-2-(N,N-diéthylaminocarbonyloxyméthyl)-pipérazine**

A une solution comprenant 1,2g (2,82 mmoles) de 1-(3',4',5'-triméthoxy-thiobenzoyl)-2-(N,N-diéthylaminocarbonyloxyméthyl)-pipérazine et 800 µl (2 équivalents) de $Et_3N$ dans 30 ml de $CH_2Cl_2$, on ajoute, par portions, 780 mg de chlorure de 3',4',5'-triméthoxybenzoyle. L'agitation est maintenue pendant une nuit à température ambiante. Après addition de 2 ml d'EtOH, la solution est lavée par 10 ml d'eau, séchée, filtrée, et évaporée. Le résidu est ensuite purifié rapidement, l'éluant étant un mélange de MeOH et de $CH_2Cl_2$ (1:99, v/v). Puis, après une cristallisation dans un mélange MeOH/diéthyl éther, on obtient 405 mg de 1-(3',4',5'-triméthoxythiobenzoyl)-4-(3',4';5'-triméthoxybenzoyl)-2-(N,N-diéthylaminocarbonyloxyméthyl)-pipérazine. Rendement : 23,2%. Rf = 0,44 ($MeOH/CH_2Cl_2$, 5:95, v/v). Point de fusion : 175,4°C.
IR : 1699 (C=O carbamate), 1642 (C=O amide), 1588 et 1507 (C=C aromatiques) $cm^{-1}$.
RMN$^1H$ (200 $MH_z$, $CDCl_3$, HMDS) δ ppm : 6,6 (s, 2H, H aromatiques), 6,6-6 (m, 2H, H aromatiques), 4,9-3,9 (m, 5H, $CH_2O$, CH et $CH_2NCS$) ; 3,8 (s, 18H, $CH_3O$), 3,7-2,6 (m, 8H, $CH_2NCO$), 1,04 (s large, 6H, $CH_3$).

**II - ACTIVITE INHIBITRICE SUR LA REPLICATION DU VIH**

L'activité inhibitrice des dérivés de la pipérazine conformes à l'Invention sur la réplication du VIH a été établie par une série d'expérimentations visant à tester *in vitro* :

- d'une part, l'aptitude de ces dérivés à inhiber la réplication du VIH dans un premier système expérimental mettant en oeuvre uniquement des cultures de macrophages dérivés des monocytes (exemple 14) ;
- d'autre part, différentes procédures de traitement de ces macrophages dérivés des monocytes par les dérivés de la pipérazine conformes à l'Invention (exemple 15) ; et
- enfin, la capacité de ces mêmes dérivés à inhiber la réplication du VIH dans deux autres systèmes expérimentaux mettant en oeuvre des cultures de monocytes non différenciés en macrophages d'une part, et des cultures de cellules mononucléées du sang périphérique d'autre part (exemple 16). Dans cette dernière population cellulaire, qui englobe les deux cibles cellulaires majeures du VIH, à savoir les macrophages et les lymphocytes T CD4+,

les différents niveaux d'activation cellulaires ont été mimés artificiellement par stimulation des cellules par l'interleukine-2 humaine recombinante après activation ou non de ces cellules par un mitogène, la phytohaemagglutinine-P.

## EXEMPLE 14 : ACTIVITE INHIBITRICE DANS LES MACROPHAGES DERIVES DES MONOCYTES

### 14. 1 - Protocole

#### a) Obtention et culture des macrophages

Les macrophages ont été obtenus par différenciation de monocytes eux-mêmes isolés de cellules mononucléées du sang périphérique.

Pour ce faire, les cellules mononucléées du sang périphérique ont été séparées des autres éléments du sang par centrifugation en gradient de ficoll (MSL 2000, Société EUROBIO) et lavées deux fois dans du milieu RPMI 1640 (Société BOEHRINGER MANNHEIM). Les monocytes ont été isolés à partir de ces cellules mononucléées par élutriation à contre-courant. Les fractions collectées ont été analysées à l'aide d'un compteur-analyseur de cellules (Société COULTER) et les cellules correspondantes à chaque fraction ont été phénotypées puis analysées à l'aide d'un cytomètre en flux (FACS, Société BECTON DICKINSON). La pureté des monocytes ainsi recueillis est au moins égale à 95%.

Ces monocytes ont été alors mis en suspension dans un milieu de culture (ci-après milieu A) comprenant du milieu RPMI 1640 (Société BOEHRINGER MANNHEIM) supplémenté par 10% de sérum de veau foetal (Société BOEHRINGER MANNHEIM) préalablement décomplémenté par un traitement à 56°C pendant 30 minutes, de la L-glutamine (2 mM) et une solution à 100 µg/ml de 3 antibiotiques : pénicilline, streptomycine, néomycine (PSN, Société LIFE TECHNOLOGIES), à raison d'1 million de monocytes par ml de milieu de culture. La suspension résultante a été répartie dans les puits de plaques de 48 puits, par dépôt d'1 ml de suspension dans chaque puits (soit 1 million de monocytes), et la différenciation des monocytes en macrophages a été obtenue en laissant les monocytes adhérer aux parois des puits pendant 7 jours. Les macrophages ont été ensuite cultivés pendant 28 jours en utilisant le même milieu de culture que celui employé pour leur différenciation.

Tout au long de leur différenciation et de leur culture ultérieure, les macrophages ont été maintenus à 37°C, dans une atmosphère saturée en humidité et comprenant 5% de $CO_2$. Dans chaque puits, le milieu de culture a été renouvelé tous les trois ou quatre jours, par prélèvement des surnageants et leur remplacement par un volume équivalent de milieu frais.

#### b) Traitement des macrophages par les dérivés de la pipérazine

Les dérivés de la pipérazine à tester ont été solubilisés dans du diméthylsulfoxyde (DMSO) à la concentration de 200 mM. Les solutions ainsi obtenues ont été aliquotées et conservées à -20°C à l'abri de la lumière. Les dilutions nécessaires à la réalisation des tests ont été préparées extemporanément dans du milieu A tel que décrit ci-avant.

Le traitement des macrophages par les dérivés de la pipérazine a été initié au troisième jour de culture (J3) - soit 24 heures avant de procéder à leur infection par le VIH (J4) -, par addition des dérivés de la pipérazine à tester au milieu de culture. Le traitement a été ensuite maintenu pendant toute la durée de la culture des macrophages, soit jusqu'au 28ème jour (J28). Pour chaque dérivé de la pipérazine, différentes doses correspondant à 0,01 µM, 0,1 µM, 1 µM, 10 µM et 100 µM ont été testées.

#### c) Infection des macrophages par le VIH

Les macrophages ont été infectés avec l'isolat de référence à tropisme macrophagique VIH-1/Ba-L (GARTNER et *al.*, *Science*, 1986, 233, 215-219) qui se réplique efficacement *in vitro* dans ce type cellulaire.

Le stock viral a été constitué en amplifiant *in vitro* la souche constitutive de cet isolat dans des cultures de cellules mononucléées du sang ombilical préalablement activées par de la phytohaemagglutinine-P (PHA-P, Société DIFCO LABORATORIES, 1 µg de PHA-P/ml de milieu de culture) et stimulées par de l'interleukine-2 humaine recombinante (IL-2, Société BOEHRINGER MANNHEIM, 20 UI d'IL-2/ml de milieu de culture). Les surnageants de ces cultures ont été centrifugés à 360 000 x g pendant 5 minutes afin d'éliminer les facteurs solubles tels que les cytokines. Les culots ont été remis en suspension dans du milieu RPMI 1640 et le stock viral a été titré au moyen de cellules mononucléées du sang périphérique préalablement activées par de la PHA-P (*Manuel des Techniques Virologiques Consensus*, Ed. ANRS). Les doses infectieuses aptes à infecter 50% des macrophages ($DI_{50}$) ont été déterminées en utilisant la formule de KÄRBER (KÄRBER et *al.*, *Arch. Exp. Path. Pharmak.*, 1931, 162, 956-959).

Les macrophages ont été infectés en introduisant dans chaque puits de culture une charge virale correspon-

dant à 10 000 $DI_{50}$, c'est-à-dire à une multiplicité d'infection égale à 0,01 (soit 0,01 particule virale/macrophage). 24 heures après avoir procédé à l'infection des macrophages, l'excès de virus a été éliminé en lavant ces macrophages à l'aide de PBS.

**d) Dosage de la réplication virale et détermination de l'inhibition de cette réplication**

La réplication virale dans les macrophages a été surveillée en dosant l'activité enzymatique de la transcriptase inverse (TI) dans les surnageants de culture tous les 3 ou 4 jours de culture, lors du remplacement de ces surnageants par du milieu frais, selon la méthode décrite par REY et *al*., *Biochem. Biophys. Res. Comm.*, 1984, 121, 126-133, et dans laquelle l'activité de la transcriptase inverse est dosée par la mesure la radioactivité incorporée lors de l'élongation du brin complémentaire d'une matrice synthétique poly-rA en présence d'une amorce oligo-$dT_{12-18}$ et d'un substrat marqué par un élément radioactif, la méthylthymidine triphosphate tritiée ($^3H$ TTP).

Pour ce faire, 400 µl du surnageant contenu dans chacun des puits de culture ont été soumis à une ultracentrifugation à 360 000 x g pendant 5 minutes. La transcriptase inverse a été libérée en procédant à une lyse du culot viral au moyen de 20 µl de NTE-Triton (NaCl 100 mM, tampon Tris 10 mM, EDTA 1 mM, Triton X-100 0,1%). Ces 20 µl ont ensuite été incubés avec 40 µl du mélange réactionnel suivant : tampon Tris 62,5 mM pH 7,8, KCl 25 mM, $MgCl_2$ 6,25 mM, DTT 1,25 mM, poly-rA et oligo-$dT_{12-18}$ 2,5.$10^{-3}$ unités de densité optique (UDO), $^3H$ TTP 5,55.$10^{-3}$ Tbq. Au bout d'1 heure, la réaction enzymatique a été arrêtée et les brins néosynthétisés ont été précipités pendant 20 minutes à 4°C par l'ajout d'lml de pyrophosphate de sodium, de 5 µg d'ADN de levure et 4 ml d'acide trichloroacétique à 20%. Le mélange résultant a été filtré à l'aide d'une membrane d'acétate de cellulose (MILLEPORE) qui retient les brins poly-dT radioactifs. Le filtre a été lavé par une solution d'acide trichloroacétique à 5%, l'eau résiduelle a été éliminée par ajout de 25 µl d'éthanol à 70% et le filtre a été séché à l'étuve pendant 10 minutes à 80°C. Ce filtre a alors été introduit dans une fiole à scintillation contenant 8 ml de liquide scintillant et la radioactivité a été déterminée au moyen d'un compteur à scintillation (Société PACKARD BELL). L'activité de la transcriptase inverse est exprimée en pmoles de méthylthymidine monophosphate tritiée ($^3H$ TMP) incorporées par heure et par ml de surnageant de culture ou plus simplement en cpm par heure et par ml de surnageant de culture.

L'inhibition de la réplication virale par les dérivés de la pipérazine a été déterminée en comparant, pour chaque dérivé testé et pour chaque dose testée de ce dérivé, l'activité de la transcriptase inverse cumulée au terme de la culture des macrophages (J28) ayant été traités par ce dérivé avec celle obtenue pour une culture de macrophages «témoins», c'est-à-dire n'ayant reçu aucun traitement par un dérivé de la pipérazine.

Pour chaque culture de macrophages traités, a été ainsi établi un pourcentage d'inhibition (I) de la réplication virale par la formule :

$$I = \frac{1 _ \text{activité TI cumulée de la culture de macrophages traités}}{\text{activité TI cumulée de la culture de macrophages "témoins"}} \times 100$$

**14. 2- Résultats**

La Figure 1 illustre, à titre d'exemple et sous la forme d'une courbe, la relation effet-dose d'un dérivé de la pipérazine conforme à l'Invention, à savoir la 1,4-di-(3',4',5'-triméthoxybenzoyl)-2-(N,N-diéthylaminocarbonyloxyméthyl)-pipérazine telle qu'obtenue à l'exemple 2, sur la réplication du VIH dans les macrophages dérivés des monocytes. Sont exprimés, en ordonnées, les pourcentages d'inhibition (I) de la réplication virale obtenus lors du traitement des macrophages par ce dérivé et, en abscisses, les doses en µM testées.

Cette Figure montre que la 1,4-di-(3',4',5'-triméthoxybenzoyl)-2-(N,N-diéthylaminocarbonyloxyméthyl)-pipérazine présente une activité inhibitrice sur la réplication du VIH dans les macrophages dérivés des monocytes dès qu'elle est utilisée à des doses supérieures à 1 µM et que sa dose efficace 50 ($DE_{50}$), c'est-à-dire la dose à laquelle elle inhibe 50% de la réplication de ce virus, est égale à 5 µM.

La 1-benzyl-4-(3',4',5'-triméthoxybenzoyl)-2-(N,N-diéthylaminocarbonyloxyméthyl)-pipérazine telle que préparée à l'exemple 8 présente une $DE_{50}$ égale à 40 µM, tandis que la 1-(3',4',5'-triméthoxybenzoyl)-4-benzyl-2-(N,N-diéthylaminocarbonyloxyméthyl)-pipérazine telle que préparée à l'exemple 10 a une $DE_{50}$ égale à 13 µM.

**EXEMPLE 15 : MISE EN OEUVRE DES DIFFERENTES PROCEDURES DE TRAITEMENT DES MACROPHAGES DERIVES DES MONOCYTES**

Différentes procédures de traitement des macrophages dérivés des monocytes par les dérivés de la pipérazine conformes à l'Invention ont été testées en utilisant un protocole similaire à celui décrit dans l'exemple 14.

Dans ces tests, toutefois, les cultures de macrophages ont été réparties en trois groupes :

- un premier groupe (ci-après groupe 1), dans lequel les macrophages ont été traités par les dérivés de la pipérazine 24 heures avant d'être infectés par le VIH (c'est-à-dire à J3) et le traitement a été maintenu jusqu'à J28, comme décrit dans l'exemple 14,
- un deuxième groupe (ci-après groupe 2) dans lequel les macrophages ont été traités 24 heures après avoir été infectés par le VIH (c'est-à-dire à J5) et le traitement a été maintenu jusqu'à J28, et
- un troisième groupe (ci-après groupe 3) dans lequel les macrophages ont été traités 24 heures avant d'être infectés par le VIH (c'est-à-dire à J3), mais le traitement n'a pas été maintenu ultérieurement.

Dans tous les cas, les dérivés de la pipérazine ont été utilisés à la dose de 100 μM et les tests ont été réalisés en triplicat.

Le Tableau 1 ci-après présente, à titre d'exemple, la moyenne des pourcentages d'inhibition (I) de la réplication virale ± écart-type obtenus en traitant, selon ces différentes procédures, les macrophages par la 1,4-di-(3',4',5'-triméthoxybenzoyl)-2-(N,N-diéthylaminocarbonyloxyméthyl)-pipérazine.

TABLEAU 1

| Groupes | I |
|---|---|
| Groupe 1 | 91 ± 2 |
| Groupe 2 | 88 ± 12 |
| Groupe 3 | 73 ± 2 |

Ces résultats montrent que la 1,4-di-(3',4',5'-triméthoxybenzoyl)-2-(N,N-diéthylaminocarbonyloxyméthyl)-pipérazine est capable d'inhiber la réplication du VIH dans les macrophages dérivés des monocytes, qu'elle soit administrée préalablement ou postérieurement à l'infection de ces macrophages par ce virus, et que son activité antivirale perdure, même en l'absence d'un traitement prolongé.

**EXEMPLE 16 : ACTIVITE INHIBITRICE DANS LES MONOCYTES NON DIFFERENCIES EN MACROPHAGES ET DANS LES CELLULES MONONUCLEES DU SANG PERIPHERIQUE**

La capacité des dérivés de la pipérazine à inhiber la réplication du VIH dans les monocytes non différenciés en macrophages et dans les cellules mononucléées du sang périphérique (CMSP) a été testée en utilisant un protocole similaire à celui décrit dans l'exemple 14.

Dans ces tests toutefois, les cultures des monocytes ont été réalisées sans étape de différenciation.

Les cultures de cellules mononucléées du sang périphérique ont été, quant à elles, réparties en trois groupes :

- un premier groupe (ci-après groupe 1) dans lequel ces cellules mononucléées ont été activées pendant les 48 premières heures de culture par de la phytohaemagglutinine-P, et stimulées tout au long de la culture par de l'interleukine-2 humaine recombinante ; ces cultures ont été réalisées dans un milieu de culture (ci-après milieu B) constitué par du milieu A supplémenté par 20 UI d'IL-2/ml de milieu de culture et l'activation des cellules mononucléées par la phytohaemagglutinine-P a été obtenue par addition d'1 μg de ce composé par ml de milieu de culture ;
- un deuxième groupe (ci-après groupe 2) dans lequel les cellules mononucléées n'ont pas été activées par la phytohaemagglutinine-P mais ont été stimulées par l'interleukine-2 humaine recombinante, et qui ont été réalisées dans du milieu B ; et
- un troisième groupe (ci-après groupe 3) dans lequel les cellules mononucléées n'ont été soumises ni à une activation par la phytohaemagglutinine-P, ni à une stimulation par l'interleukine-2 humaine recombinante, et qui ont été réalisées dans du milieu A.

Dans tous les cas, le traitement des cellules (monocytes et CMSP) par les dérivés de la pipérazine conformes à l'Invention a été initié à J3 - soit 24 heures avant de procéder à leur infection par le VIH (J4) -, par addition des dérivés de la pipérazine à tester au milieu de culture. Le traitement a été ensuite maintenu pendant toute la durée de la culture des cellules, soit jusqu'au 28ème jour (J28). Chaque dérivé de la pipérazine a été testé à la dose de 100 μM et les tests ont été réalisés en triplicat.

La Figure 2 illustre, à titre d'exemple et sous la forme d'un histogramme, la moyenne des pourcentages d'inhibition (I) de la réplication virale ± écart-type obtenus en traitant les monocytes et les cellules mononucléées du sang périphérique des groupes 1, 2 et 3 par la 1,4-di-(3',4',5'-triméthoxybenzoyl)-2-(N,N-diéthylaminocarbonyloxyméthyl)-pipérazine. Est également indiquée dans cet histogramme, la moyenne des pourcentages d'inhibition (I) de la

réplication virale ± écart-type observée en traitant des macrophages dérivés des monocytes par le même dérivé de la pipérazine et dans les mêmes conditions.

### III - CYTOTOXICITE

La cytotoxicité des dérivés de la pipérazine conformes à l'Invention a été évaluée en mesurant, au moyen de trois tests différents :

_ un test au bleu Trypan,
_ un test MTT ou test au bromure de 3-(4,5-diméthylthiazol-2-yl)-2,5-diphényltétrazolium, et
_ un test au rouge neutre,

la viabilité de cultures de cellules mononucléées du sang périphérique et de macrophages dérivés des monocytes traités par les dérivés de la pipérazine conformes à l'Invention et en comparant les résultats obtenus avec ceux observés pour des cultures «témoins», c'est-à-dire des cultures de cellules mononucléées du sang périphérique ou de macrophages dérivés des monocytes réalisées dans les mêmes conditions, mais en l'absence de tout traitement par lesdits dérivés de la pipérazine.

Le test au bleu Trypan est un test classiquement effectué sur des cellules non adhérentes et a donc été utilisé pour mesurer les effets des dérivés de la pipérazine conformes à l'Invention sur la viabilité des cellules mononucléées du sang périphérique. Ce test consiste à mélanger un échantillon de la suspension cellulaire dont on souhaite tester la viabilité, avec du bleu Trypan qui diffuse dans les cellules mortes mais pas dans les cellules vivantes, et à énumérer les cellules mortes (cellules bleues) et les cellules vivantes (cellules réfringentes) sur cellule de Malassez.

Le test MTT est, lui, au contraire classiquement effectué sur des cellules adhérentes. Il a donc été utilisé pour mesurer les effets des dérivés de la pipérazine conformes à l'Invention sur les macrophages dérivés des monocytes. Ce test consiste à incuber pendant 4 heures un échantillon de la suspension cellulaire dont on veut tester la viabilité, en présence de MTT. Le MTT, produit de couleur jaune, est métabolisé par les déshydrogénases mitochondriales des cellules vivantes en formazan qui est insoluble et de couleur violette. Ainsi, la différence d'absorbance entre le MTT et le formazan permet de quantifier, par spectrométrie, l'activité déshydrogénase des mitochondries et, par conséquent, la viabilité des cellules présentes dans la suspension (SCHWARTZ et *al.*, *AIDS Res. Hum. Retroviruses*, 1988, 4, 441-448).

Le test au rouge neutre est également un test que l'on emploie sur cellules adhérentes et qui a été utilisé pour mesurer les effets des dérivés de la pipérazine conformes à l'Invention sur les macrophages dérivés des monocytes. Ce test consiste à incuber un échantillon de la suspension cellulaire dont on veut tester la viabilité, avec du rouge neutre, lequel, contrairement au bleu Trypan, diffuse dans les cellules vivantes mais pas dans les cellules mortes, puis à éliminer l'excès de rouge neutre par lavage des cellules, puis à lyser ces dernières (par exemple au moyen d'acide acétique glacial à -20°C) et à mesurer la densité optique, par spectrophotométrie, du lysat résultant (MONTEFIORI et *al.*, *J. Clin. Microbiol.*, 1988, 26, 231-235).

Dans tous les cas, la viabilité des cellules mononucléées du sang périphérique et des macrophages dérivés des monocytes a été mesurée préalablement à leur mise en culture, puis lors de chaque renouvellement des milieux de culture.

Comme illustré à titre d'exemple par la Figure 3 qui représente, sous la forme d'un histogramme, la viabilité de cultures de macrophages dérivés des monocytes traités par la 1,4-di-(3',4',5'-triméthoxybenzoyl)-2-(N,N-diéthylaminocarbonyloxyméthyl)-pipérazine à la dose de 100 μM comparée à celle de cultures de macrophages dérivés de monocytes "témoins" telles que mesurées par le test au rouge neutre, toutes les mesures de viabilité qui ont été réalisées ont permis de mettre en évidence une absence de cytotoxicité des dérivés de la pipérazine conformes à l'Invention puisque, tout au long des cultures, on n'observe pas de différence significative dans le nombre de cellules vivantes entre les cultures de cellules traitées par ces dérivés et les cultures de cellules non traitées.

## Revendications

**1.** Utilisation d'un dérivé de la pipérazine répondant à la formule générale (I) :

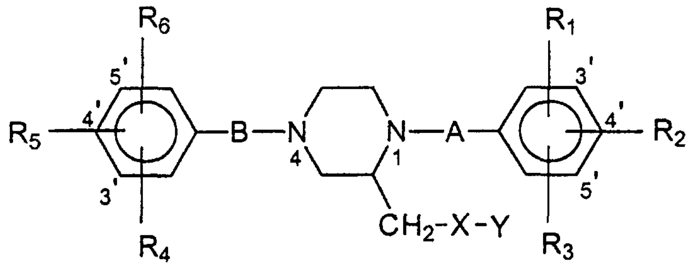

(I)

dans laquelle :

- A et B représentent, indépendamment l'un de l'autre, un groupe C=O, C=S ou $CR_7R_8$ dans lequel $R_7$ représente un atome d'hydrogène ou un groupe choisi parmi les groupes méthyle, cyano, cyanométhyle, $CO_2CH_3$ et (C=O)$CH_3$, tandis que $R_8$ représente un atome d'hydrogène ou un groupe phényle ;
- $R_1$, $R_2$, $R_3$, $R_4$, $R_5$ et $R_6$ représentent, indépendamment les uns des autres, un atome d'hydrogène, un groupe hydroxyle ou un groupe alkoxy en $C_1$ à $C_5$ linéaire ou ramifié ;
- X représente :

  - soit un groupe choisi parmi les groupes C=O, O(C=O), O(C=S), O($SO_2$), NH(C=O), NH(C=S), NH($SO_2$), S(C=O) et S(C=S), auquel cas Y représente soit un groupe $NR_9R_{10}$ ou $CR_9R_{10}R_{11}$ dans lequel $R_9$, $R_{10}$ et $R_{11}$ représentent indépendamment les uns des autres un atome d'hydrogène ou un groupe alkyle en $C_1$ à $C_5$, alcényle en $C_2$ à $C_5$ ou alcynyle en $C_2$ à $C_5$ linéaire ou ramifié, soit un hétérocycle azoté comprenant de 5 à 10 atomes et éventuellement un ou plusieurs autres hétéroatomes choisis parmi l'azote, l'oxygène et le soufre;
  - soit un atome d'oxygène ou de soufre ou un groupe choisi parmi les groupes O(C=O)O, NH(C=O)O et S(C=O)O, auquel cas Y représente un groupe $CR_9R_{10}R_{11}$ dans lequel $R_9$, $R_{10}$ et $R_{11}$ ont la même signification que précédemment ;

ou de l'un de ses sels pharmaceutiquement acceptables, pour la préparation d'un médicament pour inhiber la réplication du Virus de l'Immunodéficience Humaine (VIH).

**2.** Utilisation selon la revendication 1, **caractérisée en ce que** $R_1$, $R_2$ et $R_3$ sont portés de préférence par les atomes de carbone situés en position 3', 4' et 5' du noyau phényle lié au groupe A, tandis que $R_4$, $R_5$ et $R_6$ sont portés de préférence par les atomes de carbone situés en position 3', 4' et 5' du noyau phényle lié au groupe B.

**3.** Utilisation selon la revendication 1 ou la revendication 2, **caractérisée en ce que** le dérivé de la pipérazine répond à la formule particulière (I-a) :

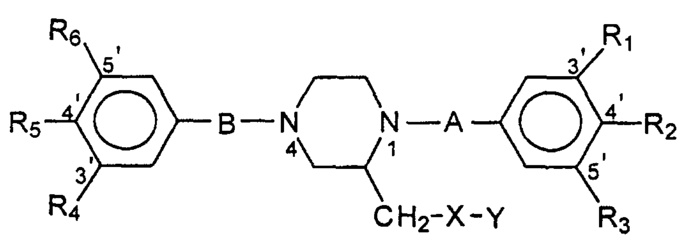

(I-a)

dans laquelle A et B représentent, indépendamment l'un de l'autre, un groupe C=O ou C=S, tandis que $R_1$, $R_2$, $R_3$, $R_4$, $R_5$, $R_6$, X et Y sont tels que définis dans la revendication 1.

**4.** Utilisation selon la revendication 3, **caractérisée en ce que**, dans la formule particulière (I-a), $R_1$, $R_2$, $R_4$ et $R_5$ représentent un groupe méthoxy, $R_3$ et $R_6$ représentent tous deux un atome d'hydrogène ou un groupe méthoxy, X représente :

- soit un groupe O(C=O) ou NH(C=O), auquel cas Y représente un groupe $NR_9R_{10}$ ou $CR_9R_{10}R_{11}$ dans lequel $R_9$, $R_{10}$ et $R_{11}$ sont tels que définis dans la revendication 1, ou bien un hétérocycle azoté comprenant de 5 à 10 atomes et éventuellement un ou plusieurs autres hétéroatomes choisis parmi l'azote, l'oxygène et le soufre ;
- soit un groupe NH(C=O)O, auquel cas Y représente un groupe $CR_9R_{10}R_{11}$ dans lequel $R_9$, $R_{10}$ et $R_{11}$ sont également tels que définis dans la revendication 1.

**5.** Utilisation selon la revendication 1 ou la revendication 2, **caractérisée en ce que** le dérivé de la pipérazine répond à la formule particulière (I-b) :

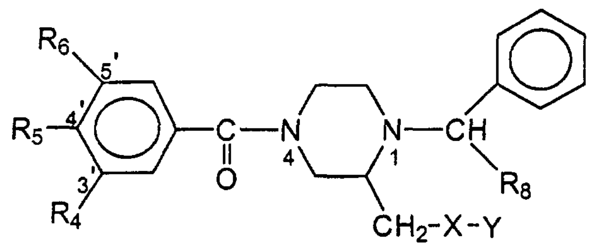

(I-b)

dans laquelle $R_8$ représente un atome d'hydrogène ou un groupe phényle, tandis que $R_4$, $R_5$, $R_6$, X et Y sont tels que définis dans la revendication 1.

**6.** Utilisation selon la revendication 5, **caractérisée en ce que**, dans la formule particulière (I-b), $R_4$, $R_5$ et $R_6$ représentent un groupe méthoxy, X représente un groupe O(C=O), tandis que Y représente un groupe $NR_9R_{10}$ dans lequel $R_9$ et $R_{10}$ représentent tous deux un groupe alkyle en $C_1$ à $C_5$, alcényle en $C_2$ à $C_5$ ou alcynyle en $C_2$ à $C_5$ linéaire ou ramifié, ou bien un hétérocycle azoté comprenant de 5 à 10 atomes et éventuellement un ou plusieurs autres hétéroatomes choisis parmi l'azote, l'oxygène et le soufre.

**7.** Utilisation selon la revendication 1 ou la revendication 2, **caractérisée en ce que** le dérivé de la pipérazine répond à la formule particulière (I-c)

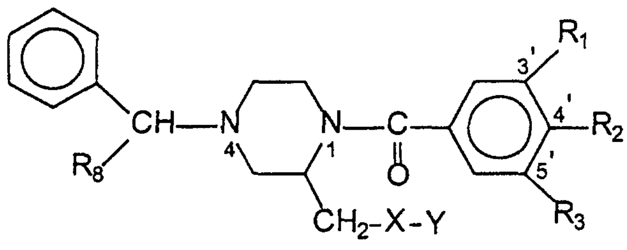

(I-c)

dans laquelle $R_8$ représente un atome d'hydrogène ou un groupe phényle, tandis que $R_1$, $R_2$, $R_3$, X et Y sont tels que définis dans la revendication 1.

**8.** Utilisation selon la revendication 7, **caractérisée en ce que**, dans la formule particulière (I-c), $R_1$, $R_2$ et $R_3$ représentent un groupe méthoxy, X représente un groupe O(C=O), tandis que Y représente soit un groupe $NR_9R_{10}$ dans lequel $R_9$ et $R_{10}$ représentent tous deux un groupe alkyle en $C_1$ à $C_5$, alcényle en $C_2$ à $C_5$ ou alcynyle en $C_2$ à $C_5$ linéaire ou ramifié, soit un hétérocycle azoté comprenant de 5 à 10 atomes et éventuellement un ou plusieurs

autres hétéroatomes choisis parmi l'azote, l'oxygène et le soufre.

**9.** Utilisation selon l'une quelconque des revendications 1 à 8, **caractérisée en ce que** le dérivé de la pipérazine est choisi parmi le groupe qui comprend les dérivés suivants :

- la 1,4-di-(3',4',5'-triméthoxybenzoyl)-2-(N,N-diéthylaminocarbonyloxyméthyl)-pipérazine,
- la 1,4-di-(3',4',5'-triméthoxybenzoyl)-2-(N,N-dipropylaminocarbonyloxyméthyl)-pipérazine,
- la 1,4-di-(3',4',5'-triméthoxybenzoyl)-2-(1'-pipéridinocarbonyloxyméthyl)-pipérazine,
- la 1,4-di-(3',4',5'-triméthoxybenzoyl)-2-(1'-pyrrolidinocarbonyloxyméthyl)-pipérazine,
- la 1,4-di-(3',4'-diméthoxybenzoyl)-2-(N,N-diéthylaminocarbonyloxyméthyl)-pipérazine,
- la 1,4-di-(3',4',5'-triméthoxybenzoyl)-2-(isopropyloxycarbonylaminométhyl)-pipérazine,
- la 1,4-di-(3',4',5'-triméthoxybenzoyl)-2-(tertiobutylcarbonylaminométhyl)-pipérazine,
- la 1-benzyl-4-(3',4',5'-triméthoxybenzoyl)-2-(N,N-diéthylaminocarbonyloxyméthyl)-pipérazine,
- la 1-diphénylméthyl-4-(3',4',5'-triméthoxybenzoyl)-2-(N,N-diéthylaminocarbonyloxyméthyl)-pipérazine, et
- la 1-(3',4',5'-triméthoxybenzoyl)-4-benzyl-2-(N,N-diéthylaminocarbonyloxyméthyl)-pipérazine,
- la 1-(3',4',5'-triméthoxybenzoyl)-4-(3',4',5'-triméthoxythiobenzoyl)-2-(N,N-diéthylaminocarbonyloxyméthyl)-pipérazine,
- la 1,4-di(3',4',5'-triméthoxythiobenzoyl)-2-(N,N- diéthylaminocarbonyloxyméthyl)-pipérazine, et
- la 1-(3',4',5'-triméthoxythiobenzoyl)-4-(3',4',5'-triméthoxybenzoyl)-2-(N,N-diéthylaminocarbonyloxyméthyl)-pipérazine.

**10.** Utilisation selon l'une quelconque des revendications 1 à 9, **caractérisée en ce que** le dérivé de la pipérazine est la 1,4-di-(3',4',5'-triméthoxybenzoyl)-2-(N,N-diéthylaminocarbonyloxyméthyl)-pipérazine.

**11.** Dérivé de la pipérazine, **caractérisé en ce qu'**il répond à la formule générale (I) telle que définie dans la revendication 1, à condition toutefois que, dans cette formule générale (I) :

_ dans le cas où $R_1$, $R_2$ et $R_3$ sont des groupes alkoxy en $C_1$ à $C_4$ saturés, linéaires ou ramifiés, et sont portés par les atomes de carbone situés en position 3', 4' et 5' du noyau phényle lié au groupe A, et où B est un groupe C=O, Y soit différent :

  - d'un groupe alkyle en $C_1$ à $C_4$, alcényle en $C_2$ à $C_4$ et alcynyle en $C_2$ à $C_4$ linéaire ou ramifié, lorsque X représente un atome d'oxygène ou de soufre ; et
  - d'un groupe alkyle en $C_1$ à $C_4$, alcényle en $C_2$ à $C_4$ et alcynyle en $C_2$ à $C_4$, linéaire ou ramifié, et d'une amine substituée ou non par un ou deux groupes alkyles en $C_1$ à $C_4$, alcényles en $C_2$ à $C_4$ et alcynyles en $C_2$ à $C_4$ linéaires ou ramifiés, lorsque X représente un groupe C=O ou O(C=O);

_ dans le cas où $R_1$, $R_2$, $R_3$, $R_4$, $R_5$ et $R_6$ représentent tous un groupe méthoxy et où $R_1$, $R_2$, $R_3$ sont portés par les atomes de carbone situés en position 3', 4' et 5' du noyau phényle lié au groupe A, tandis que $R_4$, $R_5$ et $R_6$ sont portés par les atomes de carbone situés en position 3', 4' et 5' du noyau phényle lié au groupe B, Y soit différent :

  - d'un groupe alkyle en $C_5$ ou $C_6$ linéaire ou ramifié, d'une amine substituée par un groupe alkyle en $C_5$ linéaire ou ramifié, et d'une amine substituée par deux groupes alkyles en $C_5$ linéaires, lorsque A et B représentent tous deux un groupe C=O et X représente un groupe O(C=O) ; et
  - d'un groupe $CH(CH_2CH_3)_2$, lorsque A et B représentent tous deux un groupe $CH_2$ et X représente un groupe O(C=O) ;

ainsi que ses sels pharmaceutiquement acceptables.

**12.** Dérivé de la pipérazine selon la revendication 11, **caractérisé en ce que** $R_1$, $R_2$ et $R_3$ sont portés de préférence par les atomes de carbone situés en position 3', 4' et 5' du noyau phényle lié au groupe A, tandis que $R_4$, $R_5$ et $R_6$ sont portés de préférence par les atomes de carbone situés en position 3', 4' et 5' du noyau phényle lié au groupe B.

**13.** Dérivé de la pipérazine selon la revendication 11 ou la revendication 12, **caractérisé en ce qu'**il répond à la formule particulière (I-a) :

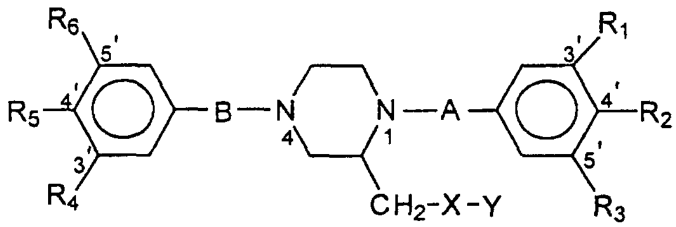

(I-a)

dans laquelle A et B représentent, indépendamment l'un de l'autre, un groupe C=O ou un groupe C=S, tandis que $R_1$, $R_2$, $R_3$, $R_4$, $R_5$, $R_6$, X et Y sont tels que définis dans la revendication 1, à condition toutefois que :

- dans le cas où $R_1$, $R_2$ et $R_3$ sont des groupes alkoxy en $C_1$ à $C_4$ saturés, linéaires ou ramifiés, Y soit différent :
  - d'un groupe alkyle en $C_1$ à $C_4$, alcényle en $C_2$ à $C_4$ et alcynyle en $C_2$ à $C_4$ linéaire ou ramifié, lorsque X représente un atome d'oxygène ou de soufre ; et
  - d'un groupe alkyle en $C_1$ à $C_4$, alcényle en $C_2$ à $C_4$ et alcynyle en $C_2$ à $C_4$ linéaire ou ramifié, et d'une amine substituée ou non par un ou deux groupes alkyles en $C_1$ à $C_4$, alcényles en $C_2$ à $C_4$ et alcynyles en $C_2$ à $C_4$ linéaires ou ramifiés, lorsque X représente un groupe C=O ou O(C=O) ;
- dans le cas où $R_1$, $R_2$, $R_3$, $R_4$, $R_5$ et $R_6$ représentent tous un groupe méthoxy, Y soit différent d'un groupe alkyle en $C_5$ ou $C_6$ linéaire ou ramifié, d'une amine substituée par un groupe alkyle en $C_5$ linéaire ou ramifié, et d'une amine substituée par deux groupes alkyles en $C_5$ linéaires, lorsque X représente un groupe O(C=O).

**14.** Dérivé de la pipérazine selon la revendication 13, **caractérisé en ce que**, dans la formule particulière (I-a), $R_1$, $R_2$, $R_4$ et $R_5$ représentent un groupe méthoxy, $R_3$ et $R_6$ représentent tous deux un atome d'hydrogène ou un groupe méthoxy, et X représente :

- soit un groupe O(C=O), auquel cas Y représente un hétérocycle azoté comprenant de 5 à 10 atomes et éventuellement un ou plusieurs autres hétéroatomes choisis parmi l'azote, l'oxygène et le soufre, ou encore, lorsque $R_3$ et $R_6$ représentent un atome d'hydrogène, un groupe $NR_9R_{10}$ dans lequel $R_9$ et $R_{10}$ représentent tous deux un groupe alkyle en $C_1$ à $C_5$, alcényle en $C_2$ à $C_5$ ou alcynyle en $C_2$ à $C_5$ linéaire ou ramifié ;
- soit un groupe NH(C=O), auquel cas Y représente soit un groupe $NR_9R_{10}$ ou $CR_9R_{10}R_{11}$ dans lequel $R_9$, $R_{10}$ et $R_{11}$ représentent indépendamment les uns des autres un atome d'hydrogène ou un groupe alkyle en $C_1$ à $C_5$, alcényle en $C_2$ à $C_5$ ou alcynyle en $C_2$ à $C_5$ linéaire ou ramifié, soit un hétérocycle azoté comprenant de 5 à 10 atomes et éventuellement un ou plusieurs autres hétéroatomes choisis parmi l'azote, l'oxygène et le soufre ;
- soit un groupe NH(C=O)O, auquel cas Y représente un groupe $CR_9R_{10}R_{11}$ dans lequel $R_9$, $R_{10}$ et $R_{11}$ sont tels que définis dans la revendication 1.

**15.** Dérivé de la pipérazine selon la revendication 11 ou la revendication 12, **caractérisé en ce qu'**il répond à la formule particulière (I-b) :

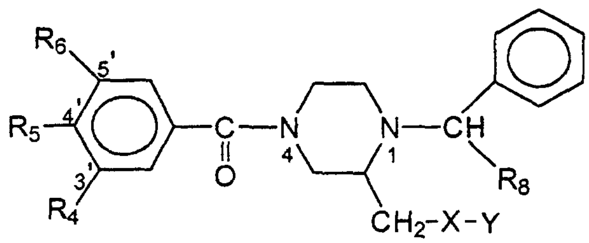

(I-b)

dans laquelle $R_8$ représente un atome d'hydrogène ou un groupe phényle, tandis que $R_4$, $R_5$, $R_6$, X et Y sont tels que définis dans la revendication 1.

**16.** Dérivé de la pipérazine selon la revendication 14, **caractérisé en ce que**, dans la formule particulière (I-b), $R_4$, $R_5$ et $R_6$ représentent un groupe méthoxy, X représente un groupe O(C=O), tandis que Y représente un groupe $NR_9R_{10}$ dans lequel $R_9$ et $R_{10}$ représentent tous deux un groupe alkyle en $C_1$ à $C_5$, alcényle en $C_2$ à $C_5$ ou alcynyle en $C_2$ à $C_5$ linéaire ou ramifié, ou bien un hétérocycle azoté comprenant de 5 à 10 atomes et éventuellement un ou plusieurs autres hétéroatomes choisis parmi l'azote, l'oxygène et le soufre.

**17.** Dérivé de la pipérazine selon la revendication 11 ou la revendication 12, **caractérisé en ce qu'**il répond à la formule particulière (I-c) :

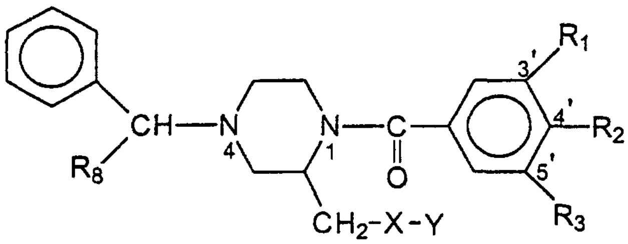

(I-c)

dans laquelle $R_8$ représente un atome d'hydrogène ou un groupe phényle, tandis que $R_1$, $R_2$, $R_3$, X et Y sont tels que définis dans la revendication 1.

**18.** Dérivé de la pipérazine selon la revendication 17, **caractérisé en ce que**, dans la formule particulière (I-c), $R_1$, $R_2$ et $R_3$ représentent un groupe méthoxy, X représente un groupe O(C=O), tandis que Y représente un groupe $NR_9R_{10}$ dans lequel $R_9$ et $R_{10}$ représentent chacun un groupe alkyle en $C_1$ à $C_5$, alcényle en $C_2$ à $C_5$ ou alcynyle en $C_2$ à $C_5$ linéaire ou ramifié, ou bien un hétérocycle azoté comprenant de 5 à 10 atomes et éventuellement un ou plusieurs autres hétéroatomes choisis parmi l'azote, l'oxygène et le soufre.

**19.** Dérivé de la pipérazine selon l'une quelconque des revendications 11 à 18, **caractérisé en ce qu'**il est l'un des composés suivants :

- la 1,4-di-(3',4',5'-triméthoxybenzoyl)-2-(1'-pipéridinocarbonyloxyméthyl)-pipérazine,
- la 1,4-di-(3',4',5'-triméthoxybenzoyl)-2-(1'-pyrrolidinocarbonyloxyméthyl)-pipérazine,
- la 1,4-di-(3',4'-diméthoxybenzoyl)-2-(N,N-diéthylaminocarbonyloxyméthyl)-pipérazine,
- la 1,4-di-(3',4',5'-triméthoxybenzoyl)-2-(isopropyloxycarbonylaminométhyl)-pipérazine,
- la 1-(3',4',5'-triméthoxybenzoyl)-4-benzyl-2-(N,N-diéthylaminocarbonyloxyméthyl)-pipérazine,
- la 1-(3',4',5'-triméthoxybenzoyl)-4-(3',4',5'-triméthoxythiobenzoyl)-2-(N,N-diéthylaminocarbonyloxyméthyl)-pipérazine, et
- la 1,4-di-(3',4',5'-triméthoxythiobenzoyl)-2-(N,N-diéthylaminocarbonyloxyméthyl)-pipérazine.

**20.** Dérivé de la pipérazine, **caractérisé en ce qu'**il est la 1-(3',4',5'-triméthoxythiobenzoyl)-4-(3',4',5'-triméthoxybenzoyl)-2-(N,N-diéthylaminocarbonyloxyméthyl)-pipérazine, et ses sels pharmaceutiquement acceptables.

**21.** Dérivé de la pipérazine selon l'une quelconque des revendications 11 à 20 et ses sels pharmaceutiquement acceptables pour l'utilisation comme médicament.

**22.** Composition pharmaceutique, **caractérisée en ce qu'**elle comprend en tant que principe actif, au moins un dérivé de la pipérazine selon l'une quelconque des revendications 11 à 20 ou l'un de ses sels pharmaceutiquement acceptables.

## Claims

**1.** Use of a piperazine derivative corresponding to the general formula (I):

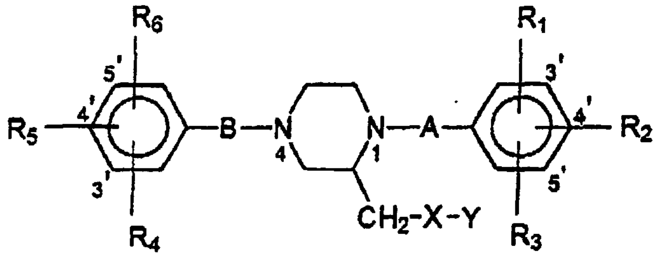

(I)

in which:

- A and B represent, independently of each other, a C=O, C=S or $CR_7R_8$ group in which $R_7$ represents a hydrogen atom or a group chosen from methyl, cyano, cyanomethyl, $CO_2CH_3$ and $(C{=}O)CH_3$ groups, while $R_8$ represents a hydrogen atom or a phenyl group;
- $R_1$, $R_2$, $R_3$, $R_4$, $R_5$ and $R_6$ represent, independently of each other, a hydrogen atom, a hydroxyl group or a linear or branched $C_1$-$C_5$ alkoxy group;
- X represents:
  - either a group chosen from C=O, O(C=O), O(C=S), $O(SO_2)$, NH(C=O), NH(C=S), $NH(SO_2)$, S(C=O) and S(C=S) groups, in which case Y represents either a group $NR_9R_{10}$ or $CR_9R_{10}R_{11}$ in which $R_9$, $R_{10}$ and $R_{11}$ represent, independently of each other, a hydrogen atom, a linear or branched $C_1$ to $C_5$ alkyl, $C_2$ to $C_5$ alkenyl or $C_2$ to $C_5$ alkynyl group, or a nitrogen heterocycle comprising from 5 to 10 atoms and optionally one or more hetero atoms chosen from nitrogen, oxygen and sulphur;
  - or an oxygen or sulphur atom or a group chosen from O(C=O)O, NH(C=O)O and S(C=O)O groups, in which case Y represents a group $CR_9R_{10}R_{11}$ in which $R_9$, $R_{10}$ and $R_{11}$ have the same meaning as above;

or one of the pharmaceutically acceptable salts thereof, for the preparation of a medicinal product for inhibiting the replication of human immunodeficiency virus (HIV).

**2.** Use according to Claim 1, **characterized in that** $R_1$, $R_2$ and $R_3$ are preferably borne by the carbon atoms in positions 3', 4' and 5' of the phenyl nucleus linked to the group A, while $R_4$, $R_5$ and $R_6$ are preferably borne by the carbon atoms in positions 3', 4' and 5' of the phenyl nucleus linked to the group B.

**3.** Use according to Claim I or Claim 2, **characterized in that** the piperazine derivative corresponds to the specific formula (I-a):

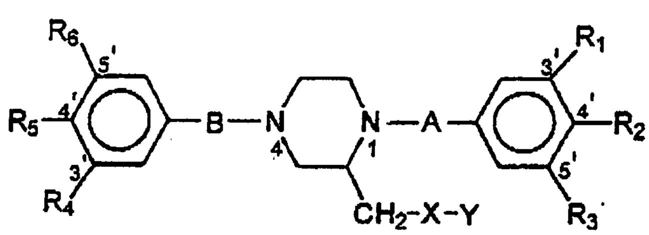

(I-a)

in which A and B represent, independently of each other, a C=O or C=S group, while $R_1$, $R_2$, $R_3$, $R_4$, $R_3$, $R_6$, X and Y are as defined in Claim 1.

**4.** Use according to Claim 3, **characterized in that**, in the specific formula (I-a), $R_1$, $R_2$, $R_4$ and $R_5$ represent a mathoxy group, $R_3$ and $R_6$ both represent a hydrogen atom or a methoxy group, X represents:

- either an O(C=O) or NH(C=O) group, in which case Y represents a group $NR_9R_{10}$ or $CR_9R_{10}R_{11}$ in which $R_9$, $R_{10}$ and $R_{11}$ are as defined in Claim 1, or alternatively a nitrogen heterocycle containing from 5 to 10 atoms and optionally one or more other hetero atoms chosen from nitrogen, oxygen and sulphur;
- or an NH(C=O)O group, in which case Y represents a group $CR_9R_{10}R_{11}$ in which $R_9$, $R_{10}$ and $R_{11}$ are also as defined in Claim 1.

**5.** Use according to Claim I or Claim 2, **characterized in that** the piperazine derivative corresponds to the specific formula (I-b):

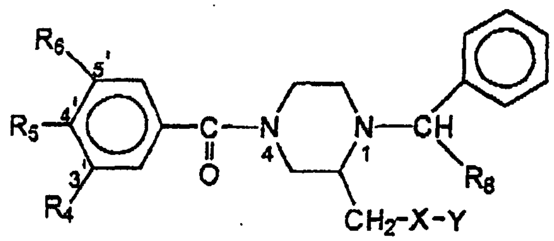

(I-b)

in which $R_8$ represents a hydrogen atom or a phenyl group, while $R_4$, $R_5$, $R_6$, X and Y are as defined in Claim 1.

**6.** Use according to Claim 5, **characterized in that**, in the specific formula (I-b), $R_4$, $R_5$ and $R_6$ represent a methoxy group, X represents an O(C=O) group, while Y represents a group $NR_9R_{10}$ in which $R_9$ and $R_{10}$ both represent a linear or branched $C_1$ to $C_5$ alkyl, $C_2$ to $C_5$ alkenyl or $C_2$ to $C_5$ alkynyl group, or alternatively a nitrogen heterocycle containing from 5 to 10 atoms and optionally one or more other hetero atoms chosen from nitrogen, oxygen and sulphur.

**7.** Use according to Claim 1 or Claim 2, **characterized in that** the piperazine derivative corresponds to the specific formula (I-c):

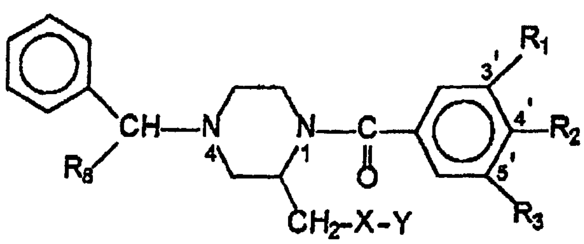

(I-c)

in which $R_8$ represents a hydrogen atom or a phenyl group, while $R_1$, $R_2$, $R_3$, X and Y are as defined in Claim 1.

**8.** Use according to Claim 7, **characterized in that**, in the specific formula (I-c), $R_1$, $R_2$ and $R_3$ represent a methoxy group, X represents an O(C=O) group, while Y represents a group $NR_9R_{10}$ in which $R_9$ and $R_{10}$ both represent a linear or branched $C_1$ to $C_5$ alkyl, $C_2$ to $C_5$ alkenyl or $C_2$ to $C_5$ alkynyl group, or a nitrogen heterocycle containing from 5 to 10 atoms and optionally one or more other hetero atoms chosen from nitrogen, oxygen and sulphur.

**9.** Use according to any one of Claims 1 to 8, **characterized in that** the piperazine derivative is chosen from the group which comprises the following derivatives:

- 1,4-bis(3',4',5'-trimethoxybenzoyl)-2-(N,N-diethylaminocarbonyloxymethyl)-piperazine,
- 1,4-bis(3',4',5'-trimethoxybenzoyl)-2-(N,N-dipropylaminocarbonyloxymethyl)-piperazine,
- 1,4-bis(3',4',5'-trimethoxybenzoyl)-2-(1'-piperidinocarbonyloxymethyl)piperazine,
- 1,4-bis(3',4',5'-trimethoxybenzoyl)-2-(1'-pyrrolidinocarbonyloxymethyl)-piperazine,
- 1,4-bis(3',4'-dimethoxybenzoyl)-2-(N,N-diethylaminocarbonyloxymethyl)-piperazine,
- 1,4-bis(3',4',5'-trimethoxybenzoyl)-2-(isopropyloxycarbonylaminomethyl)-piperazine,
- 1,4-bis(3',4',5'-trimethoxybenzoyl)-2-(tert-butylcarbonylaminomethyl)piperazine,
- 1-benzyl-4-(3',4',5'-trimethoxybenzoyl)-2-(N,N-diethylaminocarbonyloxymethyl)-piperazine,
- 1-diphenylmethyl-4-(3',4',5'-trimethoxybenzoyl)-2-(N,N-diethylaminocarbonyloxymethyl)piperazine,
- 1-(3',4',5'-trimethoxybenzoyl)-4-benzyl-2-(N,N-diethylaminocarbonyloxymethyl)piperazine,
- 1-(3',4',5'-trimethoxybenzoyl)-4-(3',4',5'-trimethoxythiobenzoyl)-2-(N,N-diethylaminocarbonyloxymethyl)piperazine,
- 1,4-bis(3',4',5'-trimethoxythiobenzoyl)-2-(N,N-diethylaminocarbonyloxymethyl)piperazine and
- 1-(3',4',5'-trimethoxythiobenzoyl)-4-(3',4',5'-trimethoxybenzoyl)-2-(N,N-diethylaminocarbonyloxymethyl)piperazine.

**10.** Use according to any one of Claims 1 to 9, **characterized in that** the piperazine derivative is 1,4-bis(3',4',5'-trimethoxybenzoyl)-2-(N,N-diethylaminocarbonyloxymethyl)piperazine.

**11.** Piperazine derivative, **characterized in that** it corresponds to the general formula (I) as defined in Claim 1, on condition, however, that, in this general formula (I):

- when $R_1$, $R_2$ and $R_3$ are saturated, linear or branched $C_1$ to $C_4$ alkoxy groups, and are borne by the carbon atoms located in positions 3', 4' and 5' of the phenyl nucleus linked to the group A, and when B is a C=O group, Y is other than:

  - a linear or branched $C_1$ to $C_4$ alkyl, $C_2$ to $C_4$ alkenyl or $C_2$ to $C_4$ alkynyl group, when X represents an oxygen or sulphur atom; and
  - a linear or branched $C_1$ to $C_4$ alkyl, $C_2$ to $C_4$ alkenyl or $C_2$ to $C_4$ alkynyl group, or an amine which is unsubstituted or substituted with one or two linear or branched $C_1$ to $C_4$ alkyl, $C_2$ to $C_4$ alkenyl or $C_2$ to $C_4$ alkynyl groups, when X represents a C=O or O(C=O) group;

- when $R_1$, $R_2$, $R_3$, $R_4$, $R_5$ and $R_6$ all represent a methoxy group and when $R_1$, $R_2$ and $R_3$ are bome by the carbon atoms located in positions 3', 4' and 5' of the phenyl nucleus linked to the group A, while $R_4$, $R_5$ and $R_6$ are borne by the carbon atoms located in positions 3', 4' and 5' of the phenyl nucleus linked to the group B, Y is other than:

  - a linear or branched $C_5$ or $C_6$ alkyl group, an amine substituted with a linear or branched $C_5$ alkyl group, or an amine substituted with two linear $C_5$ alkyl groups, when A and B both represent a C=O group and X represents an O(C=O) group; and
  - a group $CH(CH_2CH_3)_2$, when A and B both represent a $CH_2$ group and X represents an O(C=O) group;

and also the pharmaceutically acceptable salts thereof.

**12.** Piperazine derivative according to Claim 11, **characterized in that** $R_1$, $R_2$ and $R_3$ are preferably borne by the carbon atoms located in positions 3', 4' and 5' of the phenyl nucleus linked to the group A, while $R_4$, $R_5$ and $R_6$ are preferably borne by the carbon atoms located in positions 3', 4' and 5' of the phenyl nucleus linked to the group B.

**13.** Piperazine derivative according to Claim 11 or Claim 12, **characterized in that** it corresponds to the specific formula (I-a):

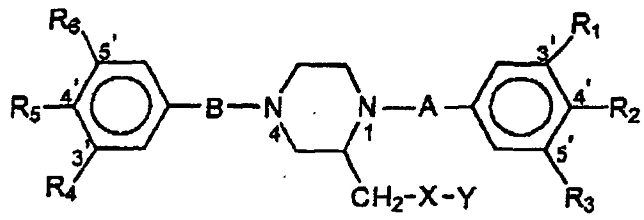

(I-a)

in which A and B represent, independently of each other, a C=O group or a C=S group, while $R_1$, $R_2$, $R_3$, $R_4$, $R_5$, $R_6$, X and Y are as defined in Claim 1, with the condition, however, that:

- when $R_1$, $R_2$ and $R_3$ are saturated, linear or branched $C_1$ to $C_4$ alkoxy groups, Y is other than:
  - a linear or branched $C_1$ to $C_4$ alkyl, $C_2$ to $C_4$ alkenyl or $C_2$ to $C_4$ alkynyl group, when X represents an oxygen or sulphur atom; and
  - a linear or branched $C_1$ to $C_4$ alkyl, $C_2$ to $C_4$ alkenyl or $C_2$ to $C_4$ alkynyl group, or an amine which is unsubstituted or substituted with one or two linear or branched $C_1$ to $C_4$ alkyl, $C_2$ to $C_4$ alkenyl or $C_2$ to $C_4$ alkynyl groups, when X represents a C=O or O(C=O) group;
- when $R_1$, $R_2$, $R_3$, $R_4$, $R_5$ and $R_6$ all represent a methoxy group, Y is other than a linear or branched $C_5$ or $C_6$ alkyl group, an amine substituted with a linear or branched $C_5$ alkyl group, or an amine substituted with two linear $C_5$ alkyl groups, when X represents an O(C=O) group.

**14.** Piperazine derivative according to Claim 13, **characterized in that**, in the specific formula (1-a), $R_1$, $R_2$, $R_4$ and $R_5$ represent a methoxy group, $R_3$ and $R_6$ both represent a hydrogen atom or a methoxy group, and X represents:

- either an O(C=O) group, in which case Y represents a nitrogen heterocycle containing from 5 to 10 atoms and optionally one or more other hetero atoms chosen from nitrogen, oxygen and sulphur, or alternatively, when $R_3$ and $R_6$ represent a hydrogen atom, a group $NR_9R_{10}$ in which $R_9$ and $R_{10}$ both represent a linear or branched $C_1$ to $C_5$ alkyl, $C_2$ to $C_5$ alkenyl or $C_2$ to $C_5$ alkynyl group;
- or an NH(C=O) group, in which case Y represents either a group $NR_9R_{10}$ or $CR_9R_{10}R_{11}$ in which $R_9$, $R_{10}$ and $R_{11}$ represent, independently of each other, a hydrogen atom or a linear or branched $C_1$ to $C_5$ alkyl, $C_2$ to $C_5$ alkenyl or $C_2$ to $C_5$ alkynyl group, or a nitrogen heterocycle containing from 5 to 10 atoms and optionally one or more other hetero atoms chosen from nitrogen, oxygen and sulphur;
- or an NH(C=O)O group, in which case Y represents a group $CR_9R_{10}R_{11}$ in which $R_9$, $R_{10}$ and $R_{11}$ are as defined in Claim 1.

**15.** Piperazine derivative according to Claim 11 or Claim 12, **characterized in that** it corresponds to the specific formula (I-b):

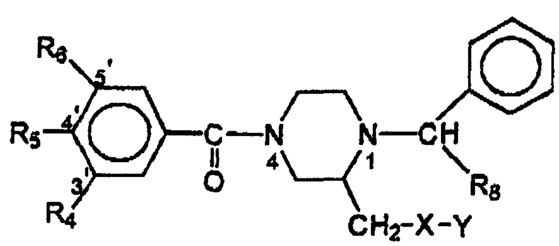

(I-b)

in which $R_8$ represents a hydrogen atom or a phenyl group, while $R_4$, $R_5$, $R_6$, X and Y are as defined in Claim 1.

**16.** Piperazine derivative according to Claim 14, **characterized in that**, in the specific formula (I-b), $R_4$, $R_5$ and $R_6$ represent a methoxy group, X represents an O(C=O) group, while Y represents a group $NR_9R_{10}$ in which $R_9$ and $R_{10}$ both represent a linear or branched $C_1$ to $C_5$ alkyl, $C_2$ to $C_5$ alkenyl or $C_2$ to $C_5$ alkynyl group, or alternatively a nitrogen heterocycle containing from 5 to 10 atoms and optionally one or more other hetero atoms chosen from nitrogen, oxygen and sulphur.

**17.** Piperazine derivative according to Claim 11 or Claim 12,
**characterized in that** it corresponds to the specific formula (I-c):

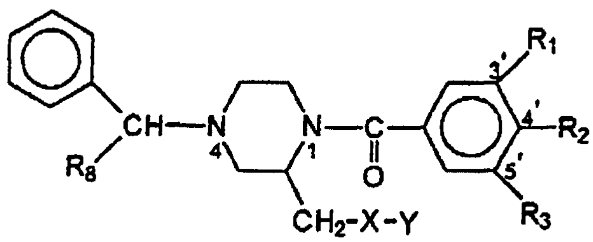

(I-c)

in which $R_8$ represents a hydrogen atom or a phenyl group, while $R_1$, $R_2$, $R_3$, X and Y are as defined in Claim 1.

**18.** Piperazine derivative according to Claim 17, **characterized in that**, in the specific formula (I-c), $R_1$, $R_2$ and $R_3$ represent a methoxy group, X represents an O(C=O) group, while Y represents a group $NR_9R_{10}$ in which $R_9$ and $R_{10}$ each represent a linear or branched $C_1$ to $C_5$ alkyl, $C_2$ to $C_5$ alkenyl or $C_2$ to $C_5$ alkynyl group, or alternatively a nitrogen heterocycle containing from 5 to 10 atoms and optionally one or more other hetero atoms chosen from nitrogen, oxygen and sulphur.

**19.** Piperazine derivative according to any one of Claims 11 to 18, **characterized in that** it is one of the following compounds:

- 1,4-bis(3',4',5'-trimethoxybenzoyl)-2-(1'-piperidinocarbonyloxymethyl)piperazine,
- 1,4-bis(3',4',5'-trimethoxybenzoyl)-2-(1'-pyrrolidinocarbonyloxymethyl)piperazine,
- 1,4-bis(3',4'-dimethoxybenzoyl)-2-(N,N-diethylaminocarbonyloxymethyl)-piperazine,
- 1,4-bis(3',4',5'-trimethoxybenzoyl)-2-(isopropyloxycarbonylaminomethyl)-piperazine,
- 1-(3',4',5'-trimethoxybenzoyl)-4-benzyl-2-(N,N-diethylaminocarbonyloxymethyl)-piperazine,
- 1-(3',4',5'-trimethoxybenzoyl)-4-(3',4',5'-trimethoxythiobenzoyl)-2-(N,N-diethylaminocarbonyloxymethyl)piperazine, and
- 1,4-bis(3',4',5'-trimethoxythiobenzoyl)-2-(N,N-diethylaminocarbonyloxymethyl)-piperazine.

**20.** Piperazine derivative, **characterized in that** it is 1-(3',4',5'-trimethoxythiobenzoyl)-4-(3',4',5'-trimethoxybenzoyl)-2-(N,N-diethylaminocarbonyloxymethyl)piperazine, and the pharmaceutically acceptable salts thereof.

**21.** Piperazine derivative according to any one of Claims 11 to 20, and the pharmaceutically acceptable salts thereof, for use as a medicinal product.

**22.** Pharmaceutical composition, **characterized in that** it comprises as active principle at least one piperazine derivative according to any one of Claims 11 to 20, or a pharmaceutically acceptable salt thereof.

## Patentansprüche

**1.** Verwendung eines Derivats von Piperazin, das der allgemeinen Formel (I) entspricht:

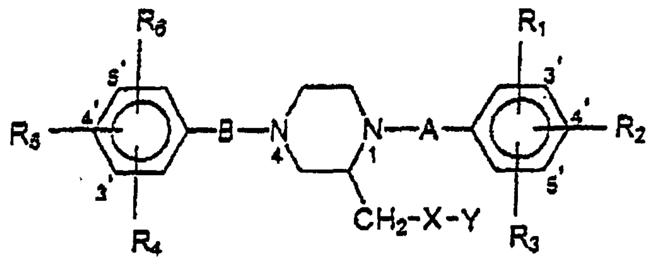

(I)

worin:

- A und B unabhängig voneinander eine Gruppe C=O, C=S oder $CR_7R_8$ darstellen, bei der $R_7$ ein Wasserstoffatom oder eine Gruppe darstellt, die aus den Gruppen Methyl, Cyano, Cyanomethyl, $CO_2CH_3$ und $(C=O)CH_3$ ausgewählt ist, während $R_8$ ein Wasserstoffatom oder eine Phenylgruppe darstellt;
- $R_1$, $R_2$, $R_3$, $R_4$, $R_5$, und $R_6$ unabhängig voneinander ein Wasserstoffatom, eine Hydroxylgruppe oder eine Alkoxylgruppe mit linearem oder verzweigtem $C_1$ bis $C_5$ darstellen;
- X folgendes darstellt:

  - entweder eine Gruppe, die aus den Gruppen C=O, O(C=O), O(C=S), $O(SO_2)$, NH(C=O), NH(C=S), $NH(SO_2)$, S(C=O) und S(C=S) ausgewählt ist, wobei Y entweder eine Gruppe $NR_9R_{10}$ oder $CR_9R_{10}R_{11}$ darstellt, bei der $R_9$, $R_{10}$ und $R_{11}$ unabhängig voneinander ein Wasserstoffatom oder eine Gruppe Alkyl mit linearem oder verzweigtem $C_1$ bis $C_5$, Alkenyl mit linearem oder verzweigtem $C_2$ bis $C_5$ oder Alkinyl mit linearem oder verzweigtem $C_2$ bis $C_5$ darstellen, oder eine stickstoffhaltige heterocyclische Verbindung, die 5 bis 10 Atome und gegebenenfalls ein oder mehrere andere Heteroatom/e umfasst, das/die aus Stickstoff, Sauerstoff und Schwefel ausgewählt ist/sind;
  - entweder ein Sauerstoff- oder Schwefelatom oder eine Gruppe, die aus den Gruppen O(C=O)O, NH(C=O)O und S(C=O)O ausgewählt ist, wobei Y eine Gruppe $CR_9R_{10}R_{11}$ darstellt, bei der $R_9$, $R_{10}$ und $R_{11}$ dieselbe Bedeutung wie oben haben;

oder eines ihrer pharmazeutisch akzeptablen Salze zur Herstellung eines Medikaments, um die Vermehrung des menschlichen Immunschwächevirus (HIV) zu hemmen.

2. Verwendung nach Anspruch 1, **dadurch gekennzeichnet, dass** $R_1$, $R_2$ und $R_3$ vorzugsweise von den an den Positionen 3', 4' und 5' angeordneten Kohlenstoffatomen des an die Gruppe A gebundenen Phenylkerns getragen werden, während $R_4$, $R_5$ und $R_6$ vorzugsweise von den an den Positionen 3', 4' und 5' angeordneten Kohlenstoffatomen des an die Gruppe B gebundenen Phenylkerns getragen werden.

3. Verwendung nach Anspruch 1 oder 2, **dadurch gekennzeichnet, dass** das Derivat von Piperazin der speziellen Formel (I-a) entspricht:

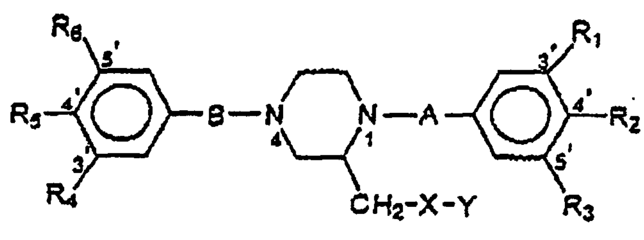

(I-a)

worin A und B unabhängig voneinander eine Gruppe C=O oder C=S darstellen, während $R_1$, $R_2$, $R_3$, $R_4$, $R_5$, $R_6$, X und Y so wie in Anspruch 1 definiert sind.

**4.** Verwendung nach Anspruch 3, **dadurch gekennzeichnet, dass** in der speziellen Formel (I-a), $R_1$, $R_2$, $R_4$, und $R_5$ eine Methoxygruppe darstellen, $R_3$ und $R_6$ beide ein Wasserstoffatom oder eine Methoxygruppe darstellen, und X folgendes darstellt:

- entweder eine Gruppe O(C=O) oder NH(C=O), wobei Y eine Gruppe $NR_9$ $R_{10}$ oder $CR_9R_{10}R_{11}$ darstellt, worin $R_9$, $R_{10}$ und $R_{11}$ so wie in Anspruch 1 definiert sind, oder aber eine stickstoffhaltige heterocyclische Verbindung, die 5 bis 10 Atome und gegebenenfalls ein oder mehrere andere Heteroatom/e umfasst, das/die aus Stickstoff, Sauerstoff und Schwefel ausgewählt ist/sind;
- oder eine Gruppe NH(C=O)O, wobei Y eine Gruppe $CR_9R_{10}R_{11}$, darstellt, bei der $R_9$,$R_{10}$ und $R_{11}$ auch so wie in Anspruch 1 definiert sind.

**5.** Verwendung nach Anspruch 1 oder 2, **dadurch gekennzeichnet, dass** das Derivat von Piperazin der speziellen Formel (I-b) entspricht:

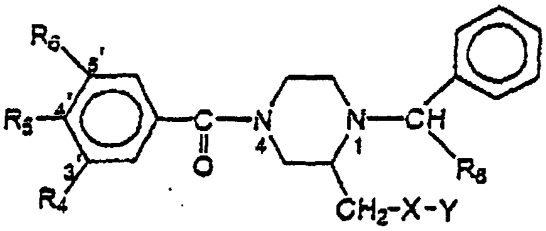

(I-b)

worin $R_8$ ein Wasserstoffatom oder eine Phenylgruppe darstellt, während $R_4$, $R_5$, $R_6$, X und Y so wie in Anspruch 1 definiert sind.

**6.** Verwendung nach Anspruch 5, **dadurch gekennzeichnet, dass** in der speziellen Formel (I-b), $R_4$, $R_5$ und $R_6$ eine Methoxygruppe darstellen, X eine Gruppe O(C=O) darstellt, während Y eine Gruppe $NR_9R_{10}$ darstellt, worin $R_9$ und $R_{10}$ beide eine Gruppe Alkyl mit linearem oder verzweigtem $C_1$ bis $C_5$, Alkenyl mit linearem oder verzweigtem $C_2$ bis $C_5$ oder Alkinyl mit linearem oder verzweigtem $C_2$ bis $C_5$ darstellen, oder eine stickstoffhaltige heterocyclische Verbindung, die 5 bis 10 Atome und gegebenenfalls ein oder mehrere andere Heteroatom/e urifasst, das/die aus Stickstoff, Sauerstoff und Schwefel ausgewählt ist/sind.

**7.** Verwendung nach Anspruch 1 oder 2, **dadurch gekennzeichnet, dass** das Derivat von Piperazin der speziellen Formel (I-c) entspricht:

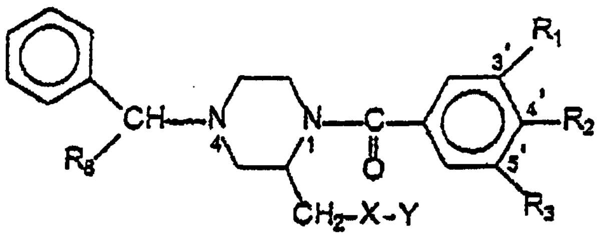

(I-c)

worin $R_8$ ein Wasserstoffatom oder eine Phenylgruppe darstellt, während $R_1$, $R_2$, $R_3$, X und Y so wie in Anspruch 1 definiert sind.

**8.** Verwendung nach Anspruch 7, **dadurch gekennzeichnet, dass** in der speziellen Formel (I-c), $R_1$, $R_2$ und $R_3$ eine Methoxygruppe darstellen, X eine Gruppe O(C=O) darstellt, während Y entweder eine Gruppe $NR_9R_{10}$ darstellt, bei der $R_9$ und $R_{10}$ beide eine Gruppe Alkyl mit linearem oder verzweigtem $C_1$ bis $C_5$, Alkenyl mit linearem oder verzweigtem $C_2$ bis $C_5$ oder Alkinyl mit linearem oder verzweigtem $C_2$ bis $C_5$ darstellen, oder eine stickstoffhaltige heterocychsche Verbindung, die 5 bis 10 Atome und gegebenenfalls ein oder mehrere andere Heteroatom/e umfasst, das/die aus Stickstoff, Sauerstoff und Schwefel ausgewählt ist/sind.

9. Verwendung nach einem der Ansprüche 1 bis 8, **dadurch gekennzeichnet, dass** das Derivat von Piperazin aus der Gruppe ausgewählt ist, die die folgenden Derivate umfasst:

- 1,4-di-(3',4',5'-Trimethoxyberuoyl)-2-(N,N-Diethylaminocarbonyloxymethyl)-Piperazin
- 1,4-di-(3',4',5'-Trimethoxybenzoyl)-2-(N,N-Dipropylaminocarbonyloxymethyl)-Piperazin
- 1,4-di-(3',4',5'-Trimethoxybenzoyl)-2-(1'-Piperidincarbonyloxymethyl)-Piperazin
- 1,4-di-(3',4',5'-Trimethoxybenzoyl)-2-(1'-Pyrrolidoncarbonyloxymethyl)-Piperazin
- 1,4-di-(3',4',5'-Dimethoxybenzoyl)-2-(N,N-Diethylaminocarbonyloxymethyl)-Piperazin
- 1,4-di-(3',4',5'-Trimethoxybenzoyl)-2-(Isopropyloxycarbonylaminomethyl)-Piperazin
- 1,4-di-(3',4',5'-Trimethoxybenzoyl)-2-(Tertiobutylcarbonylaminomethyl)-Piperazin
- 1-Benzyl-4-(3',4',5'-Trimethoxybenzoyl)-2-(N,N-Diethylaminocarbonyloxymethyl)-Piperazin
- 1-Diphenylmethyl-4-(3',4',5'-Trimethoxybenzoyl)-2-(N,N-Diethylaminocarbouyloxymethyl)-Piperazin, und
- 1-(3',4',5'-Trimethoxybenzoyl)-4-Benzyl-2-(N,N-Diethylaminocarbonyloxymethyl)-Piperazin
- 1-(3',4',5'-Trimethoxybenzoyl)-4-(3',4',5'-Trimethoxythiobenzoyl)-2-(N,N-Diethylaminocarbonyloxymethyl) -Piperazin
- 1,4-di(3',4',5'-Trimethoxythiobenzoyl)-2-(N,N-Diethylaminocarbonyloxymethyl)-Piperazin, und
- 1-(3',4',5'-Trimethoxythiobenzoyl)-4-(3',4',5'-Trimethoxybenzoyl)-2-(N,N-Diethylaminocarbonyloxymethyl) -Piperazin.

10. Verwendung nach einem der Ansprüche 1 bis 9, **dadurch gekennzeichnet, dass** das Derivat von Piperazin das 1,4-di-(3',4',5'-trimethoxybenzoyl)-2-(N,N-Diethylaminocarbonyloxymethyl)-Piperazin ist.

11. Derivat von Piperazin, **dadurch gekennzeichnet, dass** es der allgemeinen Formel (I) so wie sie in Anspruch 1 definiert ist, entspricht, jedoch unter der Bedingung, dass in dieser allgemeinen Formel (i):

- wenn $R_1$, $R_2$ und $R_3$ Alkoxygruppem mit gesättigtem, linearem oder verzweigtem $C_1$ bis $C_4$ sind, und von an den Positionen 3', 4' und 5' angeordneten Kohlenstoffatomen des an die Gruppe A gebundenen Phenylkerns getragen werden, und worin B eine Gruppe C=O ist, sich Y unterscheiden soll:

  - von einer Gruppe Alkyl mit linearem oder verzweigtem $C_1$ bis $C_4$, Alkenyl mit linearem oder verzweigtem $C_2$ bis $C_4$ und Alkinyl mit linearem oder verzweigtem $C_2$ bis $C_4$, wenn X ein Sauerstoff- oder Schwefelatom darstellt; und
  - von einer Gruppe Alkyl mit linearem oder verzweigtem $C_1$ bis $C_4$, Alkenyl mit linearem oder verzweigtem $C_2$ bis $C_4$ und Alkinyl mit linearem oder verzweigtem $C_2$ bis $C_4$, und von einem Amin, das entweder oder auch nicht durch eine oder zwei Gruppen von Alkylen mit linearem oder verzweigtem $C_1$ bis $C_4$, Alkenylen mit linearem oder verzweigtem $C_2$ bis $C_4$ und Alkinylen mit linearem oder verzweigtem $C_2$ bis $C_4$ substituiert ist, wenn X eine Gruppe C=O oder O(C=O) darstellt;

- wenn $R_1$, $R_2$, $R_3$, $R_4$, $R_5$ und $R_6$ alle eine Methoxygruppe darstellen, und wenn $R_1$, $R_2$, $R_3$ von den an den Positionen 3', 4' und 5' angeordneten Kohlenstoffatomen des an die Gruppe A gebundenen Phenylkerns getragen werden, während $R_4$, $R_5$ und $R_6$ von den an den Positionen 3', 4' und 5' angeordneten Kohlenstoffatomen des an die Gruppe B gebundenen Phenylkerns getragen werden, sich Y unterscheiden soll:

  - von einer Alkylgruppe mit linearem oder verzweigtem $C_5$ oder $C_6$, von einem durch eine Alkylgruppe mit linearem oder verzweigtem $C_5$ sunstituierten Amin, und von einem durch zwei Alkylgruppen mit linearem $C_5$ substituierten Amin, wenn A und B beide eine Gruppe C=O darstellen, und X eine Gruppe O(C=O) darstellt; und
  - von einer Gruppe $CH(CH_2CH_3)_2$, wenn A und B beide eine Gruppe $CH_2$ darstellen, und X eine Gruppe O(C=O) darstellt;

sowie seine pharmazeutisch zulässigen Salze.

12. Derivat von Piperazin nach Anspruch 11, **dadurch gekennzeichnet, dass** $R_1$, $R_2$, $R_3$ vorzugsweise von den an den Positionen 3', 4' und 5' angeordneten Kohlenstoffatomen des an die Gruppe A gebundenen Phenylkerns getragen werden, während $R_4$, $R_5$ und $R_6$ von den an den Positionen 3', 4' und 5' angeordneten Kohlenstoffatomen des an die Gruppe B gebundenen Phenylkems getragen werden.

**13.** Derivat von Piperazin nach Anspruch 11 oder 12, **dadurch gekennzeichnet, dass** es der speziellen Formel (I-a) entspricht:

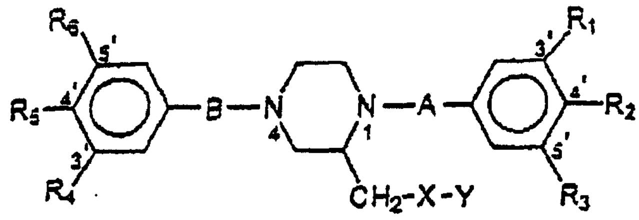

(I-a)

worin A und B unabhängig voneinander eine Gruppe C=O oder C=S darstellen, während $R_1$, $R_2$, $R_3$, $R_4$, $R_5$, $R_6$, X und Y so wie in Anspruch 1 definiert sind, jedoch unter der Bedingung, dass:

- wenn $R_1$, $R_2$ und $R_3$ Alkoxygruppem mit gesättigtem, linearem oder verzweigtem $C_1$ bis $C_4$ sind, sich Y unterscheiden soll:

  - von einer Gruppe Alkyl mit linearem oder verzweigtem $C_1$ bis $C_4$, Alkenyl mit linearem oder verzweigtem $C_2$ bis $C_4$ und Alkinyl mit linearem oder verzweigtem $C_2$ bis $C_4$, wenn X ein Sauerstoff- oder Schwefelatom darstellt; und
  - von einer Gruppe Alkyl mit linearem oder verzweigtem $C_1$ bis $C_4$, Alkenyl mit linearem oder verzweigtem $C_2$ bis $C_4$ und Alkinyl mit linearem oder verzweigtem $C_2$ bis $C_4$, und einem Amin, das entweder oder auch nicht durch eine oder zwei Gruppen von Alkylen mit linearem oder verzweigtem $C_1$ bis $C_4$, Alkenylen mit linearem oder verzweigtem $C_2$ bis $C_4$ und Alkinylen mit linearem oder verzweigtem $C_2$ bis $C_4$ substituiert ist, wenn X eine Gruppe C=O oder O(C=O) darstellt;

- wenn $R_1$, $R_2$, $R_3$, $R_4$, $R_5$ und $R_6$ alle eine Methoxygruppe darstellen, sich Y unterscheiden soll von einer Alkylgruppe mit linearem oder verzweigtem $C_5$ oder $C_6$, und von einem Amin, das durch eine Gruppe von Alkyl mit linearem oder verzweigtem $C_5$ substituiert ist, oder von einem Amin, das durch zwei Alkylgruppen mit linearem $C_5$ substituiert ist, wenn X eine Gruppe O(C=O) darstellt.

**14.** Derivat von Piperazin nach Anspruch 13, **dadurch gekennzeichnet, dass** in der speziellen Formel (I-a), $R_1$, $R_2$, $R_4$, und $R_5$ eine Methoxygruppe darstellen, $R_3$ und $R_6$ beide ein Wasserstoffatom oder eine Methoxygruppe darstellen, und X folgendes darstellt:

- entweder eine Gruppe O(C=O), wobei Y eine sdelcstofthaltige heterocyclische Verbindung darstellt, die 5 bis 10 Atome und gegebenenfalls ein oder mehrere andere Heteroatom/c umfasst, das/die aus Stickstoff, Sauerstoff und Schwefel ausgewählt ist/sind oder aber, wenn $R_3$ und $R_6$ ein Wasserstoffatom darstellen, eine Gruppe $NR_9R_{10}$, wobei $R_9$ und $R_{10}$ beide eine Gruppe Alkyl mit linearem oder verzweigtem $C_1$ bis $C_5$, Alkenyl mit linearem oder verzweigtem $C_2$ bis $C_5$ oder Alkinyl mit linearem oder verzweigtem $C_2$ bis $C_5$ darstellen;
- oder eine Gruppe NH(C=O), wobei Y eine Gruppe $NR_9R_{10}$ oder $CR_9R_{10}R_{11}$ darstellt, bei der $R_9$, $R_{10}$ und $R_{11}$ unabhängig voneinander ein Wasserstoffatom oder eine Gruppe Alkyl mit linearem oder verzweigtem $C_1$ bis $C_5$, Alkenyl mit linearem oder verzweigtem $C_2$ bis $C_5$ oder Alkinyl mit linearem oder verzweigtem $C_2$ bis $C_5$ darstellen, oder eine stickstoffhaltige heterocyclische Verbindung, die 5 bis 10 Atome und gegebenenfalls ein oder mehrere andere Heteroatom/e umfasst, das/die aus Stickstoff, Sauerstoff und Schwefel ausgewählt ist/sind;
- oder eine Gruppe NH(C=O)O, wobei Y eine Gruppe $CR_9R_{10}R_{11}$ darstellt, bei der $R_9$, $R_{10}$ und $R_{11}$ so wie in Anspruch 1 definiert sind.

**15.** Derivat von Piperazin nach Anspruch 11 oder 12, **dadurch gekennzeichnet, dass** es der speziellen Formel (I-b) entspricht:

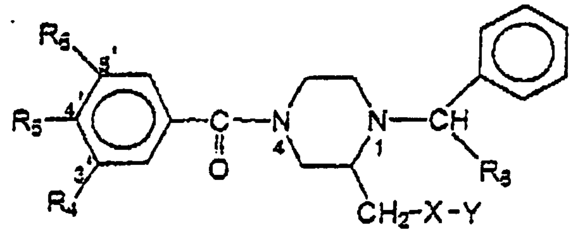

(I-b)

worin $R_8$ ein Wasserstoffatom oder eine Phenylgruppe darstellt, wahrend $R_4$, $R_5$, $R_6$, X und Y so wie in Anspruch 1 definiert sind.

**16.** Derivat von Piperazin nach Anspruch 14, **dadurch gekennzeichnet, dass** in der speziellen Formel (I-b), $R_4$, $R_5$ und $R_6$ eine Methoxygruppe darstellon, X eine Gruppe O(C=O) darstellt, während Y eine Gruppe $NR_9R_{10}$ darstellt, bei der $R_9$ und $R_{10}$ beide eine Gruppe Alkyl mit linearem oder verzweigtem $C_1$ bis $C_5$, Alkenyl mit linearem oder verzweigtem $C_2$ bis $C_5$ oder Alkinyl mit linearem oder verzweigtem $C_2$ bis $C_5$ darstellen, oder eine stickstoffhaltige heterocyclische Verbindung, die 5 bis 10 Atome und gegebenenfalls ein oder mehrere andere Heteroatom/e umfasst, die aus Stickstoff, Sauerstoff und Schwefel ausgewählt ist/sind.

**17.** Derivat von Piperazin nach Anspruch 11 oder 12, **dadurch gekennzeichnet, dass** es der speziellen Formel (I-c) entspricht:

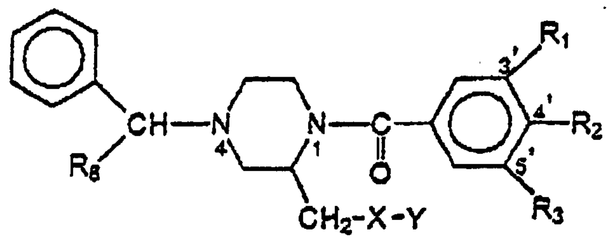

(I-c)

worin $R_8$ ein Wasserstoffatom oder eine Phenylgruppe darstellt, während $R_1$, $R_2$, $R_3$, X und Y so wie in Anspruch 1 definiert sind.

**18.** Derivat von Piperazin nach Anspruch 17, **dadurch gekennzeichnet, dass** in der speziellen Formel (I-c), $R_1$, $R_2$ und $R_3$ eine Methoxygruppe darstellen, X eine Gruppe O(C=O) darstellt, während Y eine Gruppe $NR_9R_{10}$ darstellt, bei der $R_9$ und $R_{10}$ beide eine Gruppe Alkyl mit linearem oder verzweigtem $C_1$ bis $C_5$, Alkenyl mit linearem oder verzweigtem $C_2$ bis $C_5$ oder Alkinyl mit linearem oder verzweigtem $C_2$ bis $C_5$ darstellen, oder eine stickstoffhaltige heterocyclische Verbindung, die 5 bis 10 Atome und gegebenenfalls ein oder mehrere andere Heteroatom/e umfasst, das/die aus Stickstoff. Sauerstoff und Schwefel ausgewählt ist/sind.

**19.** Derivat von Piperazin nach einem der Ansprüche 11 bis 18, **dadurch gekennzeichnet, dass** es einer der folgenden Bestandteile ist:

- 1,4-di-(3',4',5'-Trimethoxybenzoyl)-2-(1'-Piperidincarbonyloxymethyl)-Piperazin
- 1,4-di-(3',4',5'-Trimethoxybenzoyl)-2-(1'-Pyrrolidoncarbonyloxymethyl)-Piperazin
- 1,4-di-(3',4' -Dimethoxybenzoyl)-2-(N,N-Diethylaminocarbonyloxymethyl)-Piperazin
- 1,4-di-(3',4',5'-Trimethoxybenzoyl)-2-(Isopropyloxycarbonylaminomethyl)-Piperazin
- 1-(3',4',5'-Trimethoxybenzoyl)-4-Benzyl-2-(N,N-Diethylaminocarbonyloxymethyl)-Piperazin
- 1-(3',4',5'-Trimethoxybenzoyl)-4-(3',4',5'-Trimethoxythiobenzoyl)-2-(N,N-Diethylaminocarbonyloxymethyl) -Piperazin, und
- 1,4-di(3',4',5'-Trimethoxythiobenzoyl)-2-(N,N-Diethylaminocarbonyloxymethyl)-Piperazin.

**20.** Derivat von Piperazin, **dadurch gekennzeichnet, dass** es 1-(3',4',5'-Trimethoxythiobenzoyl)-4-(3',4',5'-Trimethoxybenzoyl)-2-(N,N-Diethylaminocarbonyloxymethyl)-Piperazin und seine pharmazeutisch zulässigen Salze sind.

**21.** Derivat von Piperazin nach einem der Ansprüche 11 bis 20 und seine pharmazeutisch zulässigen Salze zur Verwendung als Medikament.

**22.** Pharmazeutische Zusammensetzung, **dadurch gekennzeichnet, dass** sie als Wirkstoff mindestens ein Derivat von Piperazin nach einem der Ansprüche 11 bis 20 oder eines seiner pharmazeutisch zulässigen Salze aufweist.

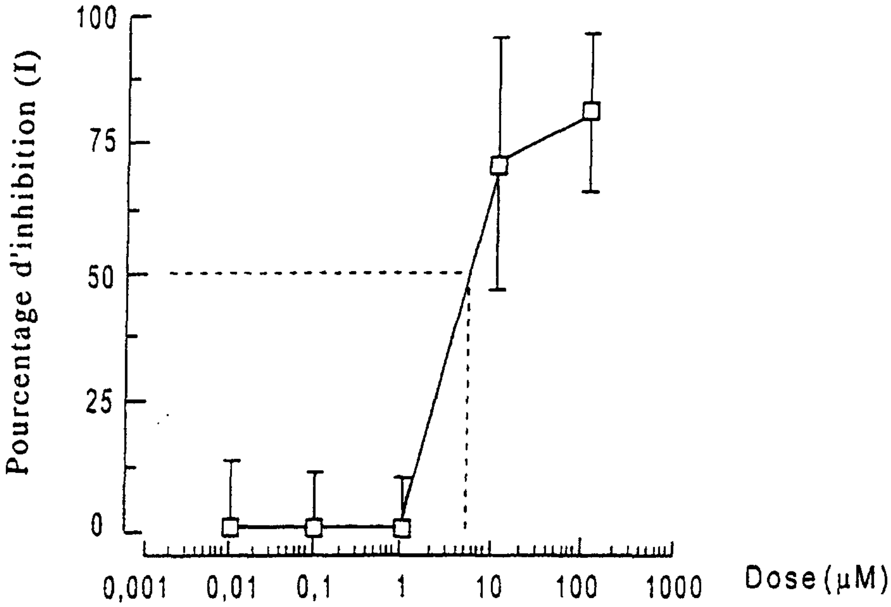
Pourcentage d'inhibition (I)
100
75
50
25
0
0,001
0,01
0,1
1
10
100
1000
Dose (μM)

FIG. 1

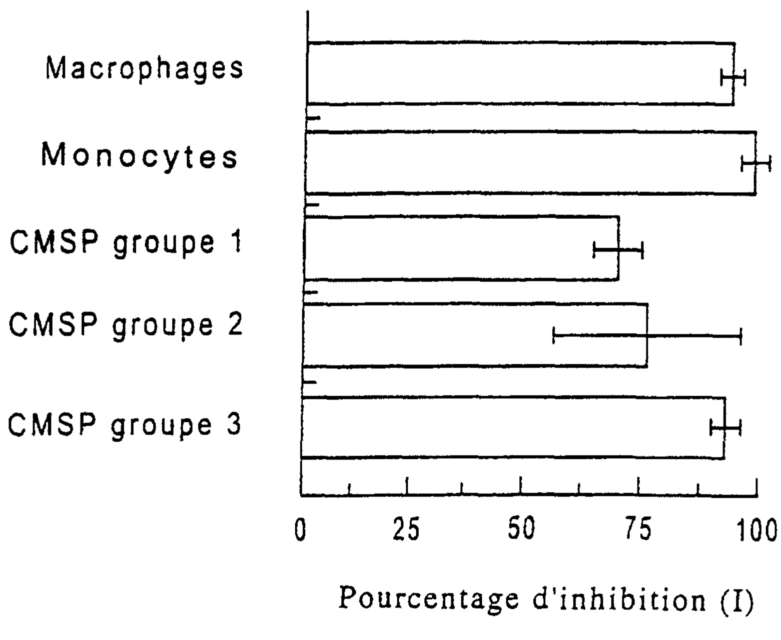
Macrophages
Monocytes
CMSP groupe 1
CMSP groupe 2
CMSP groupe 3
0
25
50
75
100
Pourcentage d'inhibition (I)

FIG. 2

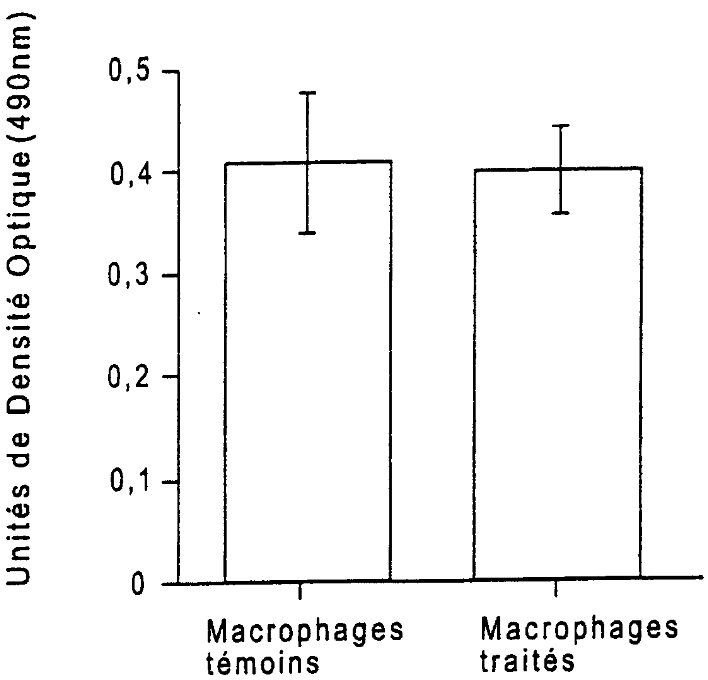
Unités de Densité Optique (490nm)
0,5
0,4
0,3
0,2
0,1
0
Macrophages témoins
Macrophages traités

FIG. 3